# EUROPEAN PATENT SPECIFICATION

(11) **EP 4 519 235 B1**
(45) Date of publication and mention of the grant of the patent: **09.12.2026**
(21) Application number: 23723246.7
(22) Date of filing: 04.05.2023
(51) Int. Cl.: C07C 37/84, A61K 31/00, A61K 31/05, A61P 25/08, C07C 39/23

(54) **CRYSTALINE FORMS OF A CANNABIDIOL-LIKE CANNABINOID**
KRISTALLINE FORMEN EINES CANNABIDIOLÄHNLICHEN CANNABINOIDS
FORMES CRISTALLINES D'UN CANNABINOÏDE DE TYPE CANNABIDIOL

(30) Priority: 04.05.2022 GB 202206506
(43) Date of publication of application: 12.03.2025
(73) Proprietor: Jazz Pharmaceuticals Research UK Limited, Sittingbourne Kent ME9 8AG (GB)
(72) Inventor: STRAKER, Hannah E, Sittingbourne Kent, ME9 8AG (GB); MCMILLAN, Lynn, Sittingbourne Kent, ME9 8AG (GB); TAYLOR, Louise, Sittingbourne Kent, ME9 8AG (GB); MCKENNA, Laura, Sittingbourne Kent, ME9 8AG (GB)
(74) Representative: HGF
(86) International application number: PCT/GB2023/051181
(87) International publication number: WO 2023/214172

(56) References cited:
- WO-A1-2021/099783

## Description

### RELATED APPLICATIONS

The present application is related to, and claims the benefit of, GB 2206506.4 filed on 04 May 2022 (04.05.2022).

### FIELD OF THE INVENTION

The present invention relates to a crystalline form of 6-hydroxy cannabidivarin (6-OH CBDV), methods for their production, and their use in therapy.

### BACKGROUND TO THE INVENTION

Alpha 6-hydroxy cannabidivarin (6-OH CBDV) is an oxidised cannabidiol-like (CBD-like) cannabinoid derivative having the formula:

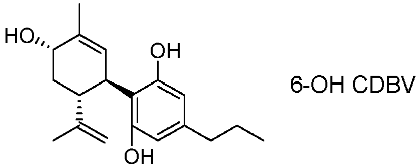

International application PCT/GB2020/052944 provides data demonstrating the efficacy of 6-OH CBDV in two mouse models of seizure. Thus, 6-OH CBDV will be useful in the treatment of epilepsy.

PCT/GB2020/052944 describes the production of 6-OH CBDV by reduction of 6-oxo-CBDV diacetate. The 6-OH CBDV product was purified by column chromatography to provide glassy (amorphous) solid or semi-solid material. WO 2021/099783A1 describes the chemical synthesis of 6-OH CBDV from cannabidivarin (CBDV). The final product is obtained as a white glassy solid. This document discloses that 6-OH CBDV has anticonvulsant activity in a MES and MEST test with mice.

The present invention has been devised in light of these considerations.

### BRIEF SUMMARY OF THE INVENTION

At its most general, the present invention relates to 6-OH CBDV in crystalline form. The crystalline form is easier to handle than glassy, amorphous material.

In a first aspect of the invention there is provided 6-OH CBDV in crystalline form.

In a second aspect of the invention, there is provided crystalline Form A 6-OH CBDV. The inventors have found that Form A has excellent chemical stability. For example, Form A is particularly stable to various stress conditions including compression and milling, which aids processing and handling during formulation of a pharmaceutical product. Form A also has a high melting-point, further improving processability

Form A 6-OH CBDV is characterized by an X-ray powder diffraction (XRPD) pattern comprising peaks at 7.83, 12.50, 13.01, 14.64, 18.30, 20.79, 23.90, 24.60, 25.43, and 26.22 ±0.2 (°2θ).

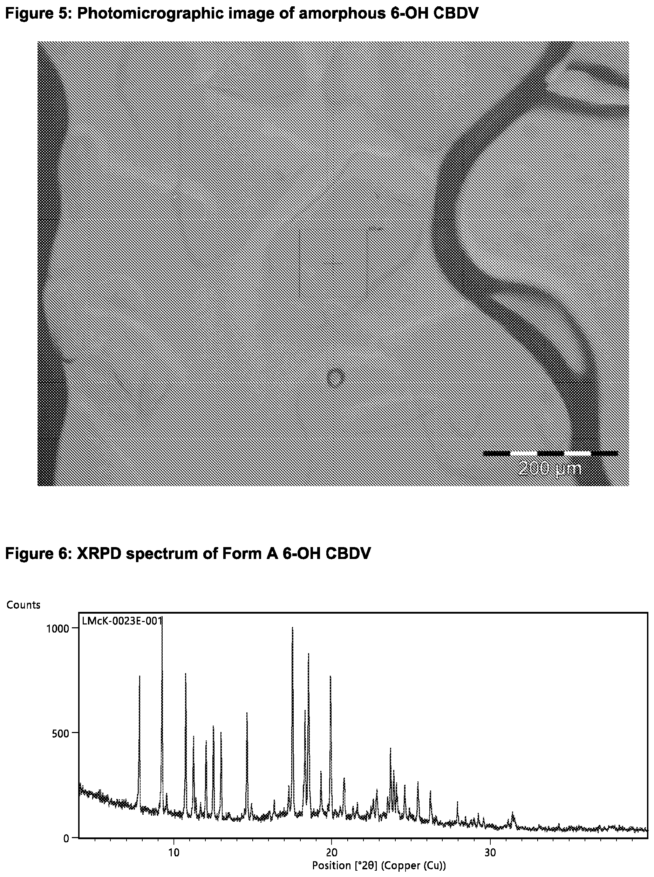

Form A material may have a differential scanning calorimetry (DCS) thermogram comprising a peak with an onset temperature (Tₒₙₛₑₜ) of between 142 °C and 153 °C, such as about 151 °C. Form A material may comprise rod-like crystals. Form A material may have an XRPD pattern further comprising peaks at 9.26, 10.75, 11.24, 12.03, 17.28, 18.52, 23.70, and 24.08 ±0.2 (°2θ). Further peaks may also occur at 17.52, 19.31, and 19.94 ±0.2 (°2θ). Form A material may have an XRPD pattern substantial as shown in Figure 6. Form A material may be anhydrous.

In a third aspect of the present disclosure, there is provided crystalline Form B 6-OH CBDV. Form B is highly ordered (low disorder).

Form B 6-OH CBDV is characterized by an X-ray powder diffraction (XRPD) pattern comprising peaks at 9.44, 10.10, 13.95, 15.89, 16.31, 20.51, 21.17, 21.76 and 26.21 ±0.2 (°2θ).

Form B material may have a thermogravimetric differential thermal analysis (TG/DTA) thermogram comprising a peak with a Tₒₙₛₑₜ of about 78 ±2 °C. Form B may be a methyl ethyl ketone solvate.

In a fourth aspect of the present disclosure, there is provided crystalline Form C 6-OH CBDV.

Form C 6-OH CBDV is characterized by an X-ray powder diffraction (XRPD) pattern comprising peaks at 9.59, 10.20, 13.96, 14.22, 15.85, 16.14, 16.51, 20.53, 20.92, 21.40, 21.86, 24.39, and 24.65 ±0.2 (°2θ).

Form C material may have a TG/DTA thermogram comprising a peak with a Tₒₙₛₑₜ of about 80 ±2 °C. Form C material may be an acetone solvate.

In a fifth aspect of the present disclosure, there is provided crystalline Form D 6-OH CBDV.

Form D 6-OH CBDV is characterized by an X-ray powder diffraction (XRPD) pattern comprising peaks at 8.16, 12.46, 22.24, and 26.77 ±0.2 (°2θ).

Form D material may have a TG/DTA thermogram comprising a peak with a Tₒₙₛₑₜ of about 94 °C.

In a sixth aspect of the present disclosure, there is provided crystalline Form E 6-OH CBDV.

Form E 6-OH CBDV is characterized by an X-ray powder diffraction (XRPD) pattern comprising peaks at 10.73, 10.96, 12.62, 15.37, 17.52, 18.68, and 19.74 ±0.2 (°2θ).

Form E material may have a TG/DTA thermogram comprising a peak with a Tₒₙₛₑₜ of about 117 °C. Form E material may be a dimethylacetamide solvate.

In a seventh aspect of the invention or present disclosure, there is provided a composition comprising 6-OH CBDV, wherein 5 wt% or more, such as 50 wt% or more, 90 wt% or more, or 95 wt% or more of the 6-OH CBDV is the crystalline form of the second, third, fourth, fifth, or sixth aspects.

In an eight aspect of the invention or present disclosure, there is provided a pharmaceutical composition comprising the crystalline form of any of the first, second, third, fourth, fifth, or sixth aspects, or the composition the seventh aspect, together with one or more ingredients selected from carriers, diluents, excipients, adjuvants, fillers, buffers, binders, disintegrants, preservatives, antioxidants, lubricants, stabilisers, solubilisers, surfactants, masking agents, colouring agents, flavouring agents, and sweetening agents.

Preferably, the pharmaceutical composition may be in a form selected from a tablet, a granule, a powder, a capsule, and a pill.

In a ninth aspect of the invention or present disclosure, there is provided the crystalline form of any of the first, second, third, fourth, fifth, or sixth aspects; the composition the seventh aspect; or the pharmaceutical composition of the eight aspect, for use in a method of treatment.

Preferably, the method of treatment of the ninth aspect is a method of treatment of epilepsy.

In a tenth aspect of the invention, there is provided a method for preparing a crystalline form of 6-OH CBDV, preferably Form A 6-OH CBDV, the method comprising the following steps:
(a) providing a composition comprising 6-OH CBDV and a solvent selected from water and a hydrocarbon solvent;
(b) heating the composition;
(c) cooling the composition; and
(d) isolating the solids.

These and other aspects and embodiments of the invention are described in further detail below.

### BRIEF SUMMARY OF THE DRAWINGS

The present invention is described with reference to the figures listed below:
**Figure 1** is an XRPD spectrum of amorphous 6-OH CBDV.
**Figure 2** is a TG/DTA thermogram of amorphous 6-OH CBDV.
**Figure 3** is a DSC thermogram of amorphous 6-OH CBDV.
**Figure 4** is a hyper DSC thermogram of amorphous 6-OH CBDV.
**Figure 5** is a photomicrographic image of amorphous 6-OH CBDV.
**Figure 6** is an XRPD spectrum of Form A 6-OH CBDV.
**Figure 7** is a TG/DTA thermogram of Form A 6-OH CBDV.
**Figure 8** is a DSC thermogram of Form A 6-OH CBDV.
**Figure 9** is a photomicrographic image of Form A 6-OH CBDV.
**Figure 10** shows the XRPD pattern of Form A material before (top) and after (bottom) compression.
**Figure 11** is an XRPD spectrum of Form B 6-OH CBDV.
**Figure 12** is a TG/DTA thermogram of Form B 6-OH CBDV (after drying under vacuum for approx.. 2 hr).
**Figure 13** is an XRPD spectrum of Form C 6-OH CBDV.
**Figure 14** is a TG/DTA thermogram of Form C 6-OH CBDV.
**Figure 15** is an XRPD spectrum of Form D 6-OH CBDV.
**Figure 16** is a TG/DTA thermogram of Form D 6-OH CBDV.
**Figure 17** is an XRPD spectrum of Form E 6-OH CBDV.
**Figure 18** is a TG/DTA thermogram of Form E 6-OH CBDV.
**Figure 19** shows the XRPD spectrum of Form E (pink; top) and Form E after drying under vacuum for approx. 2 hr (blue; bottom).
**Figure 20** shows the TG/DTA thermogram of Form E 6-OH CBDV (after drying under vacuum for approx. 2 hr).

### DETAILED DESCRIPTION OF THE INVENTION

The references to methods of treatment in the subsequent paragraphs of this description are to be interpreted as references to the compounds, pharmaceutical compositions and medicaments of the present invention for use in a method for the treatment of the human or animal body by therapy.

The present invention relates provides a crystalline form of 6-OH CBDV.

Characterisation of a crystalline material, such as a crystalline form of 6-OH CBDV, and differentiation of a crystalline material from an amorphous material can be achieved using known techniques. In particular, verification of the form can be achieved using techniques such as melting point, differential scanning calorimetry, thermogravimetric differential thermal analysis, optical microscopy and X-ray powder diffraction.

### Form A

In one aspect, the present invention provides crystalline Form A 6-OH CBDV. Crystalline Form A material is particularly preferred because it has excellent chemical stability. Form A displays good stability to compression and milling, which aids processing and handling during formulation of a pharmaceutical product. Form A also has a high melting-point, further improving processability.

The XRPD pattern of crystalline Form A 6-OH CBDV displays characteristic peaks (see, e.g., Figure 6). The XRPD pattern of the material may be determined using standard techniques, such as using a Panalytical Empyrean diffractometer equipped with a Cu X-ray tube and a PIXcel 1D-Medipix3 detector system. Sample analysis may be performed at, for example, ambient temperature in transmission mode, with a spin rate of 60 rpm. Characteristic peaks may be identified based on their position (°2θ). Characteristic peaks may be further identified by their relative intensity in comparison to the largest identified peak using a suitable cut-off value (for example, a relative intensity of greater than 15%).

In one embodiment, the Form A 6-OH CBDV is characterised by an XRPD pattern comprising at least one peak at a position (°2θ) selected from 7.83, 12.50, 13.01, 14.64, 18.30, 20.79, 23.90, 24.60, 25.43, and 26.22 ±0.2, such as measured using Cu-K_{α} irradiation (1.54060 Å).

Preferably, the Form A 6-OH CBDV is characterised by an XRPD pattern comprising at least two peaks, more preferably at least three peaks, even more preferably at least five peaks, and most preferably at least seven peaks at positions (°2θ) selected from 7.83, 12.50, 13.01, 14.64, 18.30, 20.79, 23.90, 24.60, 25.43, and 26.22 ±0.2, such as measured using Cu-K_{α} irradiation (1.54060 Å).

In one embodiment, the Form A 6-OH CBDV is characterised by an XRPD pattern comprising peaks at 7.83, 12.50, 13.01, 14.64, 18.30, 20.79, 23.90, 24.60, 25.43, and 26.22 ±0.2 (°2θ), such as measured using Cu-K_{α} irradiation (1.54060 Å).

In one embodiment, the Form A 6-OH CBDV is characterised by an XRPD pattern further comprising peaks at 9.26, 10.75, 11.24, 12.03, 17.28, 18.52, 23.70, and 24.08 ±0.2 (°2θ), such as measured using Cu-K_{α} irradiation (1.54060 Å).

In one embodiment, the Form A 6-OH CBDV is characterised by an XRPD pattern further comprising peaks at 17.52, 19.31, and 19.94 ±0.2 (°2θ), such as measured using Cu-K_{α} irradiation (1.54060 Å).

In one embodiment, the Form A 6-OH CBDV has an XRPD pattern substantial as shown in Figure 6.

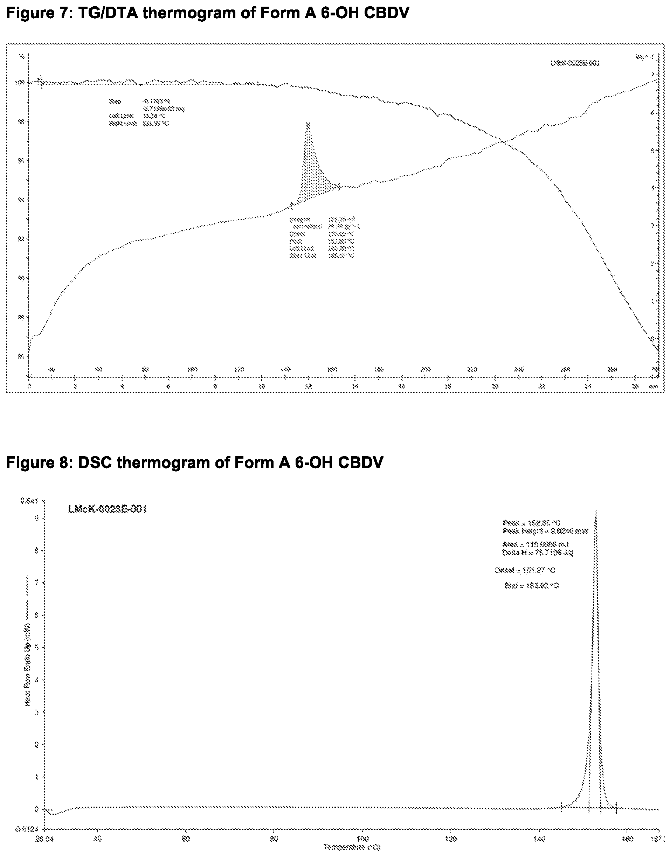

The DSC thermogram of crystalline Form A 6-OH CBDV displays characteristic peaks (Figure 8). The DCS thermogram may be determined using standard techniques, such as using a Perkin Elmer Jade Differential Scanning Calorimeter with a heating rate of 10°C/minute to a maximum of 235°C.

In one embodiment, the Form A 6-OH CBDV is characterised by a DSC thermogram comprising a peak with an onset temperature (Tₒₙₛₑₜ) of between 142 °C and 153 °C, such as measured at a heating rate of 10 °C/min. Preferably, the Form A 6-OH CBDV is characterised by a DSC thermogram comprising a peak with Tₒₙₛₑₜ of 151 ±2 °C, such as measured at a heating rate of 10 °C/min.

In one embodiment, the Form A 6-OH CBDV has a DSC thermogram substantial as shown in Figure 8.

In one embodiment, the Form A 6-OH CBDV comprises rod-like crystals.

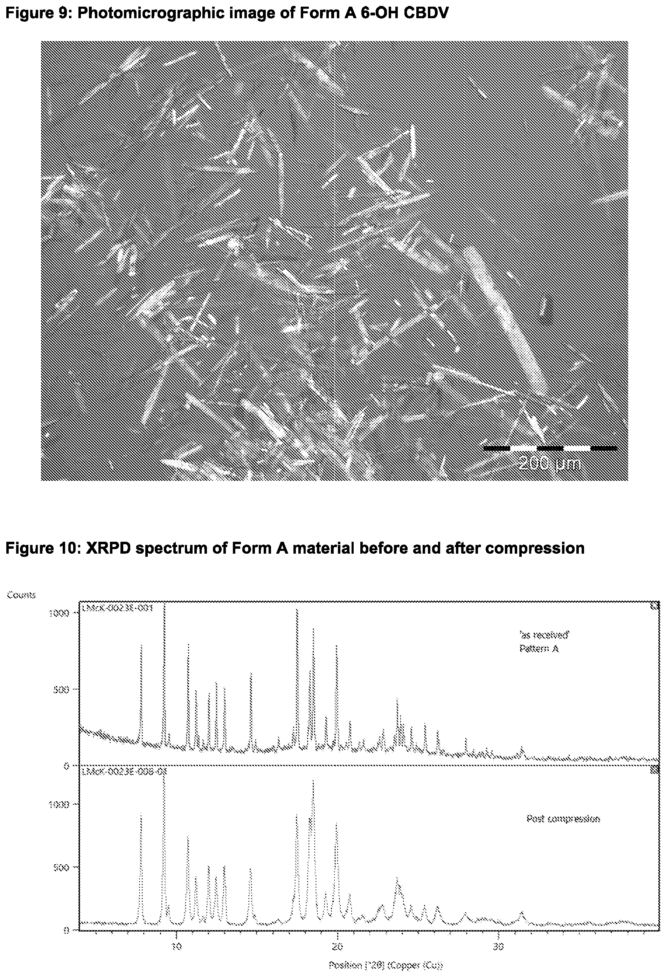

In one embodiment, the Form A 6-OH CBDV is substantially as shown in Figure 9.

In one embodiment, the Form A 6-OH CBDV is anhydrous (does not contain bound water). In a preferred embodiment the Form A 6-OH CBDV does not contain any bound solvent.

In one aspect, the invention provides a composition comprising 6-OH CBDV, wherein 5 wt% or more of the 6-OH CBDV is Form A 6-OH CBDV. In a preferred embodiment, the invention provides a composition comprising 6-OH CBDV, wherein 10 wt% or more, more preferably 50 wt% or more, of the 6-OH CBDV is Form A 6-OH CBDV.

In another aspect, the invention provides a composition comprising 6-OH CBDV, wherein 85 wt% or more of the 6-OH CBDV is Form A 6-OH CBDV. In a preferred embodiment, the invention provides a composition comprising 6-OH CBDV, wherein 90 wt% or more, more preferably 95 wt% or more, even more preferably 98 wt% or more, of the 6-OH CBDV is Form A 6-OH CBDV.

In one aspect, the invention provides a method for preparing crystalline Form A 6-OH CBDV, the method comprising the following steps:
(a) providing a composition comprising 6-OH CBDV and a solvent selected from water and a hydrocarbon solvent;
(b) heating the composition;
(c) cooling the composition; and
(d) isolating the solids.

The 6-OH CBDV used in the preparation step (step a) may be any form of 6-OH CBDV. Typically, the 6-OH CBDV used in the preparation method is or comprises amorphous 6-OH CBDV.

The hydrocarbon solvent may be an aliphatic or aromatic hydrocarbon solvent.

Examples of suitable aliphatic hydrocarbon solvents include linear alkanes such as pentane, hexane, heptane and octane; cycloalkanes such as cyclopentane, cyclohexane, cycloheptane and cyclooctane; and petroleum fractions such as kerosene and petroleum ether. Examples of suitable aromatic hydrocarbon solvents include benzene, toluene and xylene.

In one embodiment, the organic solvent is an aliphatic hydrocarbon solvent, preferably the organic solvent is heptane or pentane.

In a preferred embodiment, the organic solvent is an aromatic hydrocarbon solvent, more preferably the organic solvent is toluene.

The heating step (step b) may be carried out to a predetermined maximum temperature. Typically, the predetermined maximum temperature is the lower of i) 10 °C lower than the solvent boiling point, preferably 5 °C or 3°C lower than the solvent boiling point; or ii) 100°C.

The heating step (step b) may be carried out at a predetermined heating rate. Typically, the predetermined heating rate is from 0.2 °C/min to 20 °C/min, preferably from 0.2 °C/min to 10 °C/min, or 0.5 °C/min to 5 °C/min.

The cooling step (step c) may be carried out to a predetermined minimum temperature. Typically, the predetermined minimum temperature is ambient temperature (~20 °C).

The cooling step (step c) may be carried out at a predetermined cooling rate. Typically, the predetermined heating rate is from 0.05 °C/min to 10 °C/min, preferably from 0.05 °C/min to 5 °C/min, or 0.1 °C/min to 2 °C/min.

The isolation step (step d) may comprise isolating the solid material by any suitable method. Suitable methods include filtration and centrifugation.

Optionally, the method for preparing crystalline Form A 6-OH CBDV comprising repeated heating and cooling cycles (e.g. repeating steps b and c). In one embodiment, the preparation method may comprise 2 or 3 repetitions.

In another aspect, the invention provides a method for preparing crystalline Form A 6-OH CBDV, the method comprising the following steps:
(a) providing a composition comprising 6-OH CBDV and a solvent selected from water and a hydrocarbon solvent;
(b) heating the composition;
(c) seeding the composition with crystalline Form A 6-OH CBDV;
(d) cooling the composition; and
(e) isolating the solids.

Preferences for the steps (a), (b), (d) and (e) are set out above.

In one aspect, the invention provides crystalline Form A 6-OH CBDV prepared by the method outline in paragraphs [0050] to [0061] or [0062] to [0063].

### Form B

In one aspect, the present disclosure provides crystalline Form B 6-OH CBDV.

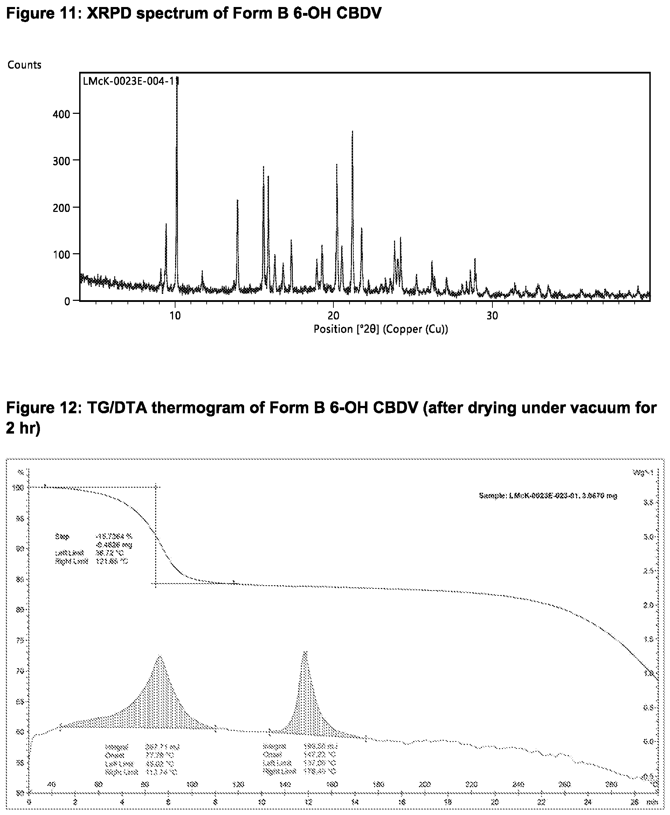

The XRPD pattern of crystalline Form B 6-OH CBDV displays characteristic peaks (see, e.g., Figure 11). The XRPD pattern of the material may be determined using standard techniques (e.g., as set out for Form A, above).

In one embodiment, the Form B 6-OH CBDV is characterised by an XRPD pattern comprising at least one peak at a position (°2θ) selected from 9.44, 10.10, 13.95, 15.89, 16.31, 20.51. 21.17, 21.76, and 26.21 ±0.2, such as measured using Cu-K_{α} irradiation (1.54060 Å).

Preferably, the Form B 6-OH CBDV is characterised by an XRPD pattern comprising at least two peaks, more preferably at least three peaks, even more preferably at least five peaks, and most preferably at least seven peaks at positions (°2θ) selected from 9.44, 10.10, 13.95, 15.89, 16.31, 20.51. 21.17, 21.76, and 26.21 ±0.2, such as measured using Cu-K_{α} irradiation (1.54060 Å).

In one embodiment, the Form B 6-OH CBDV is characterised by an XRPD pattern comprising peaks at 9.44, 10.10, 13.95, 15.89, 16.31, 20.51. 21.17, 21.76, and 26.21 ±0.2 (°2θ), such as measured using Cu-K_{α} irradiation (1.54060 Å).

In one embodiment, the Form B 6-OH CBDV is characterised by an XRPD pattern further comprising peaks at 15.58, 17.33, 23.84, and 24.23 ±0.2 (°2θ), such as measured using Cu-K_{α} irradiation (1.54060 Å).

In one embodiment, the Form B 6-OH CBDV is characterised by an XRPD pattern further comprising peaks at 19.26 and 20.19 ±0.2 (°2θ), such as measured using Cu-K_{α} irradiation (1.54060 Å).

In one embodiment, the Form B 6-OH CBDV has an XRPD pattern substantial as shown in Figure 11.

The TG/DTA thermogram of crystalline Form B 6-OH CBDV displays characteristic peaks (Figure 12). The TG/DTA thermogram may be determined using standard techniques, such as using a Mettler Toledo TGA/DSC1 STARe using iridium and tin as calibration standards and with a heating rate of 10°C/minute to a maximum of 300°C.

In one embodiment, the Form B 6-OH CBDV is characterised by a TG/DTA thermogram comprising a peak with an onset temperature (Tₒₙₛₑₜ) of from 78 ±2 °C, such as measured at a heating rate of 10 °C/min. Typically, the onset temperature is associated with a weight loss of from 10 wt% to 20 wt%, such as 14 wt% to 18 wt%, such as about 15.7 wt%.

In one embodiment, the Form B 6-OH CBDV is characterised by a TG/DTA thermogram comprising a peak with an onset temperature (Tₒₙₛₑₜ) of 147 ±2 °C, such as measured at a heating rate of 10 °C/min.

In one embodiment, the Form B 6-OH CBDV has a TG/DTA thermogram substantial as shown in Figure 12.

In one embodiment, the Form B 6-OH CBDV is an MEK (methyl ethyl ketone) solvate, such as an MEK mono-solvate.

In one aspect, the present disclosure provides a composition comprising 6-OH CBDV, wherein 5 wt% or more of the 6-OH CBDV is Form B 6-OH CBDV. In a preferred embodiment, the present disclosure provides a composition comprising 6-OH CBDV, wherein 10 wt% or more, more preferably 50 wt% or more, of the 6-OH CBDV is Form B 6-OH CBDV.

In another aspect, the present disclosure provides a composition comprising 6-OH CBDV, wherein 85 wt% or more of the 6-OH CBDV is Form B 6-OH CBDV. In a preferred embodiment, the present disclosure provides a composition comprising 6-OH CBDV, wherein 90 wt% or more, more preferably 95 wt% or more, even more preferably 98 wt% or more, of the 6-OH CBDV is Form B 6-OH CBDV.

In one aspect, the present disclosure provides a method for preparing crystalline Form B 6-OH CBDV, the method comprising the following steps:
(a) providing a composition comprising 6-OH CBDV and MEK;
(b) evaporating the solvent; and
(c) isolating the solids.

The 6-OH CBDV used in the preparation step (step a) may be any form of 6-OH CBDV. Typically, the 6-OH CBDV used in the preparation method is or comprises amorphous 6-OH CBDV.

The evaporation step (step b) may be carried out at any suitable temperature. Typically, the evaporation step is carried out at from 15 °C to 30°C, such as about 20 °C.

In one aspect, the present disclosure provides crystalline Form B 6-OH CBDV prepared by the method outline in paragraphs [0080] to [0082].

### Form C

In one aspect, the present disclosure provides crystalline Form C 6-OH CBDV.

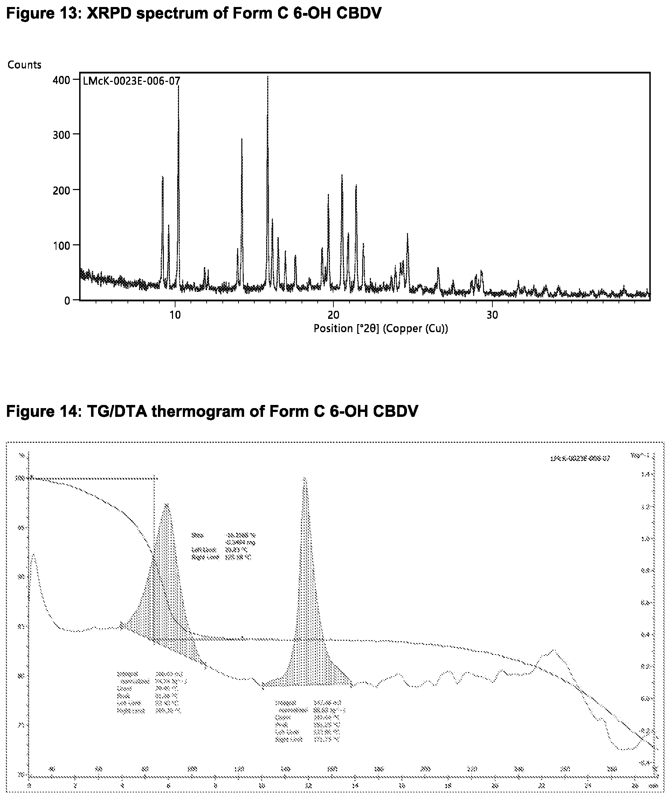

The XRPD pattern of crystalline Form C 6-OH CBDV displays characteristic peaks (see, e.g., Figure 13). The XRPD pattern of the material may be determined using standard techniques (e.g., as set out for Form A, above).

In one embodiment, the Form C 6-OH CBDV is characterised by an XRPD pattern comprising at least one peak at a position (°2θ) selected from 9.59, 10.20, 13.96, 14.22, 15.85, 16.14, 16.51, 20.53, 20.92, 21.40, 21.86, 24.39, and 24.65 ±0.2, such as measured using Cu-K_{α} irradiation (1.54060 Å).

Preferably, the Form C 6-OH CBDV is characterised by an XRPD pattern comprising at least two peaks, more preferably at least three peaks, even more preferably at least five peaks, and most preferably at least seven peaks at positions (°2θ) selected from 9.59, 10.20, 13.96, 14.22, 15.85, 16.14, 16.51, 20.53, 20.92, 21.40, 21.86, 24.39, and 24.65 ±0.2, such as measured using Cu-K_{α} irradiation (1.54060 Å).

In one embodiment, the Form C 6-OH CBDV is characterised by an XRPD pattern comprising peaks at 9.59, 10.20, 13.96, 14.22, 15.85, 16.14, 16.51, 20.53, 20.92, 21.40, 21.86, 24.39, and 24.65 ±0.2 (°2θ), such as measured using Cu-K_{α} irradiation (1.54060 Å).

In one embodiment, the Form C 6-OH CBDV is characterised by an XRPD pattern further comprising peaks at 9.21, 16.97, and 24.22 ±0.2 (°2θ), such as measured using Cu-K_{α} irradiation (1.54060 Å).

In one embodiment, the Form C 6-OH CBDV is characterised by an XRPD pattern further comprising peaks at 17.58, 19.27, and 19.67 ±0.2 (°2θ), such as measured using Cu-K_{α} irradiation (1.54060 Å).

In one embodiment, the Form C 6-OH CBDV has an XRPD pattern substantial as shown in Figure 13.

The TG/DTA thermogram of crystalline Form C 6-OH CBDV displays characteristic peaks (Figure 14). The TG/DTA thermogram may be determined using standard techniques(e.g., as set out for Form B, above).

In one embodiment, the Form C 6-OH CBDV is characterised by a TG/DTA thermogram comprising a peak with an onset temperature (Tₒₙₛₑₜ) of 80 ±2 °C, such as measured at a heating rate of 10 °C/min. Typically, the onset temperature is associated with a weight loss of from 10 wt% to 20 wt%, such as 14 wt% to 18 wt%, such as about 16.3 wt%.

In one embodiment, the Form C 6-OH CBDV is characterised by a TG/DTA thermogram comprising a peak with an onset temperature (Tₒₙₛₑₜ) of 148 ±2 °C, such as measured at a heating rate of 10 °C/min.

In one embodiment, the Form C 6-OH CBDV has a TG/DTA thermogram substantial as shown in Figure 14.

In one embodiment, the Form C 6-OH CBDV is an acetone solvate, such as an acetone mono-solvate.

In one aspect, the present disclosure provides a composition comprising 6-OH CBDV, wherein 5 wt% or more of the 6-OH CBDV is Form C 6-OH CBDV. In a preferred embodiment, the present disclosure provides a composition comprising 6-OH CBDV, wherein 10 wt% or more, more preferably 50 wt% or more, of the 6-OH CBDV is Form C 6-OH CBDV.

In another aspect, the present disclosure provides a composition comprising 6-OH CBDV, wherein 85 wt% or more of the 6-OH CBDV is Form C 6-OH CBDV. In a preferred embodiment, the present disclosure provides a composition comprising 6-OH CBDV, wherein 90 wt% or more, more preferably 95 wt% or more, even more preferably 98 wt% or more, of the 6-OH CBDV is Form C 6-OH CBDV.

In one aspect, the present disclosure provides a method for preparing crystalline Form C 6-OH CBDV, the method comprising the following steps:
(a) providing a first vessel comprising 6-OH CBDV;
(b) exposing the 6-OH CBDV to an atmosphere comprising water and acetone; and
(c) isolating the solids.

The 6-OH CBDV used in the preparation step (step a) may be any form of 6-OH CBDV. Typically, the 6-OH CBDV used in the preparation method is or comprises amorphous 6-OH CBDV.

The vapour stress step (step b) may be carried out by providing a second container comprising water and acetone, such as a 1:4 v/v acetone:water mixture, and allowing communication of the atmosphere (the vapours) between the two vessels. For example, the first vessel may be placed inside the second vessel.

The vapour stress step (step b) may be carried out at any suitable temperature. Typically, the vapour stress step is carried out at from 15 °C to 30°C, such as about 20 °C.

In one aspect, the present disclosure provides crystalline Form C 6-OH CBDV prepared by the method outline in paragraphs [0099] to [0102].

### Form D

In one aspect, the present disclosure provides crystalline Form D 6-OH CBDV.

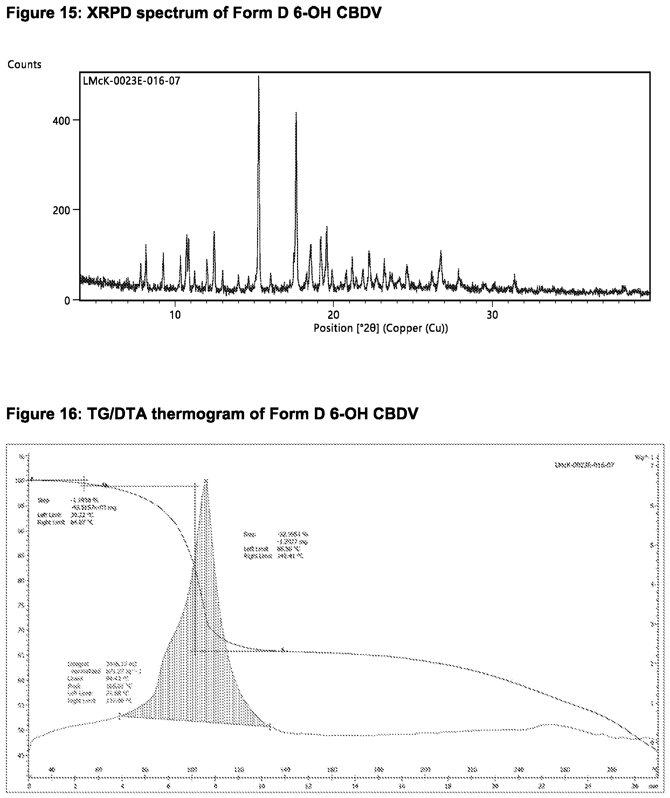

The XRPD pattern of crystalline Form D 6-OH CBDV displays characteristic peaks (see, e.g., Figure 15). The XRPD pattern of the material may be determined using standard techniques (e.g., as set out for Form A, above).

In one embodiment, the Form D 6-OH CBDV is characterised by an XRPD pattern comprising at least one peak at a position (°2θ) selected from 8.16, 12.46, 22.24, and 26.77 ±0.2, such as measured using Cu-K_{α} irradiation (1.54060 Å).

Preferably, the Form D 6-OH CBDV is characterised by an XRPD pattern comprising at least two peaks, more preferably at least three peaks at positions (°2θ) selected from 8.16, 12.46, 22.24, and 26.77 ±0.2, such as measured using Cu-K_{α} irradiation (1.54060 Å).

In one embodiment, the Form D 6-OH CBDV is characterised by an XRPD pattern comprising peaks at 8.16, 12.46, 22.24, and 26.77 ±0.2 (°2θ), such as measured using Cu-K_{α} irradiation (1.54060 Å).

In one embodiment, the Form D 6-OH CBDV is characterised by an XRPD pattern further comprising peaks at 9.25, 10.73, 10.85, 12.00, 15.28, 18.56 ±0.2 (°2θ), such as measured using Cu-K_{α} irradiation (1.54060 Å).

In one embodiment, the Form D 6-OH CBDV is characterised by an XRPD pattern further comprising peaks at 17.65, 19.19, and 19.56 ±0.2 (°2θ), such as measured using Cu-K_{α} irradiation (1.54060 Å).

In one embodiment, the Form D 6-OH CBDV has an XRPD pattern substantial as shown in Figure 15.

The TG/DTA thermogram of crystalline Form D 6-OH CBDV displays characteristic peaks (Figure 16). The TG/DTA thermogram may be determined using standard techniques (e.g., as set out for Form B, above).

In one embodiment, the Form D 6-OH CBDV is characterised by a TG/DTA thermogram comprising a peak with an onset temperature (Tₒₙₛₑₜ) of 94 ±2 °C, such as measured at a heating rate of 10 °C/min. Typically, the onset temperature is associated with a weight loss of from 25 wt% to 40 wt%, such as 30 wt% to 35 wt%, such as about 33 wt%.

In one embodiment, the Form D 6-OH CBDV has a TG/DTA thermogram substantial as shown in Figure 16.

In one aspect, the present disclosure provides a composition comprising 6-OH CBDV, wherein 5 wt% or more of the 6-OH CBDV is Form D 6-OH CBDV. In a preferred embodiment, the present disclosure provides a composition comprising 6-OH CBDV, wherein 10 wt% or more, more preferably 50 wt% or more, of the 6-OH CBDV is Form D 6-OH CBDV.

In another aspect, the present disclosure provides a composition comprising 6-OH CBDV, wherein 85 wt% or more of the 6-OH CBDV is Form D 6-OH CBDV. In a preferred embodiment, the present disclosure provides a composition comprising 6-OH CBDV, wherein 90 wt% or more, more preferably 95 wt% or more, even more preferably 98 wt% or more, of the 6-OH CBDV is Form D 6-OH CBDV.

In one aspect, the present disclosure provides a method for preparing crystalline Form D 6-OH CBDV, the method comprising the following steps:
(a) providing a composition comprising 6-OH CBDV and a solvent comprising NMP and water;
(b) sonicating the composition; and
(c) isolating the solids.

The 6-OH CBDV used in the preparation step (step a) may be any form of 6-OH CBDV. Typically, the 6-OH CBDV used in the preparation method is or comprises crystalline Form A 6-OH CBDV.

The solvent used in the preparation step (step a) may comprise a ratio of NMP:water of from 5:95 to 30:70, such as 10:90 (v/v).

The sonication step (step b) may be carried out intermittently, such as in 30 second intervals. Typically, the sonication step is carried out using a sonication intensity of from 75 W to 100 W, such as from 80 W to 95 W, such as about 91 W.

In one aspect, the present disclosure provides crystalline Form D 6-OH CBDV prepared by the method outline in paragraphs [0117] to [0120].

### Form E

In one aspect, the present disclosure provides crystalline Form E 6-OH CBDV.

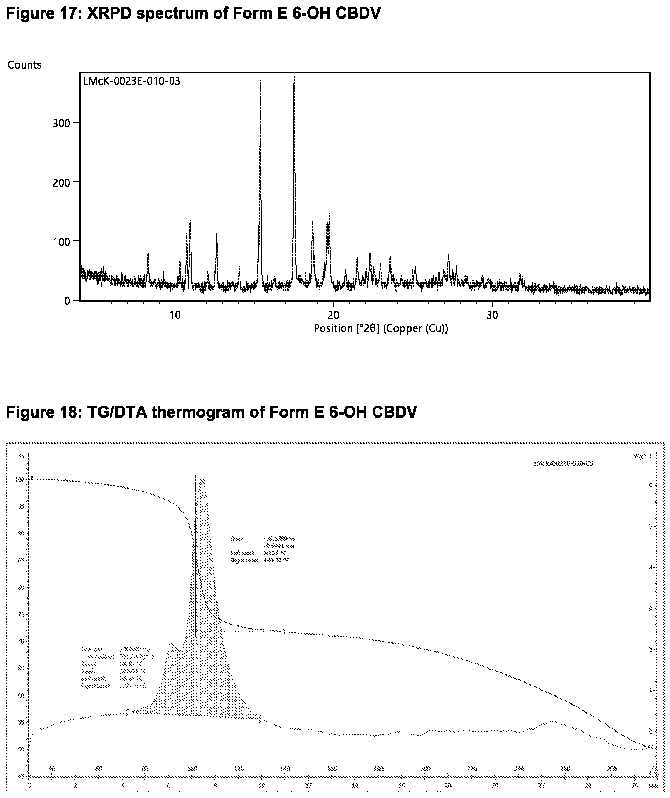

The XRPD pattern of crystalline Form E 6-OH CBDV displays characteristic peaks (see, e.g., Figure 17). The XRPD pattern of the material may be determined using standard techniques (e.g., as set out for Form A, above).

In one embodiment, the Form E 6-OH CBDV is characterised by an XRPD pattern comprising at least one peak at a position (°2θ) selected from 10.73, 10.96, 12.62, 15.37, 17.52, 18.68, and 19.74 ±0.2, such as measured using Cu-K_{α} irradiation (1.54060 Å).

Preferably, the Form E 6-OH CBDV is characterised by an XRPD pattern comprising at least two peaks, more preferably at least three peaks, even more preferably at least five peaks at positions (°2θ) selected from 10.73, 10.96, 12.62, 15.37, 17.52, 18.68, and 19.74 ±0.2, such as measured using Cu-K_{α} irradiation (1.54060 Å).

In one embodiment, the Form E 6-OH CBDV is characterised by an XRPD pattern comprising peaks at 10.73, 10.96, 12.62, 15.37, 17.52, 18.68, and 19.74 ±0.2 (°2θ), such as measured using Cu-Kα irradiation (1.54060 Å).

In one embodiment, the Form E 6-OH CBDV has an XRPD pattern substantial as shown in Figure 17.

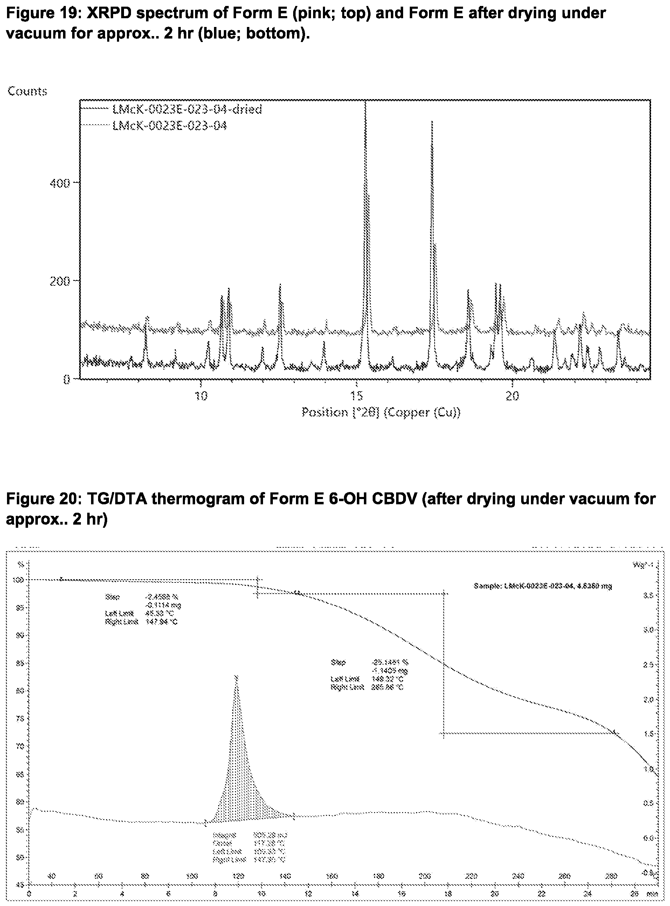

The TG/DTA thermogram of crystalline Form E 6-OH CBDV displays characteristic peaks (Figure 20). The TG/DTA thermogram may be determined using standard techniques (e.g., as set out for Form B, above).

In one embodiment, the Form E 6-OH CBDV is characterised by a TG/DTA thermogram comprising a peak with an onset temperature (Tₒₙₛₑₜ) of 117 ±2 °C, such as measured at a heating rate of 10 °C/min.

In one embodiment, the Form E 6-OH CBDV has a TG/DTA thermogram substantial as shown in Figure 20.

In one embodiment, the Form E 6-OH CBDV is a DMAc (dimethylacetamide) solvate, such as an DMAc mono-solvate.

In one aspect, the present disclosure provides a composition comprising 6-OH CBDV, wherein 5 wt% or more of the 6-OH CBDV is Form E 6-OH CBDV. In a preferred embodiment, the present disclosure provides a composition comprising 6-OH CBDV, wherein 10 wt% or more, more preferably 50 wt% or more, of the 6-OH CBDV is Form E 6-OH CBDV.

In another aspect, the present disclosure provides a composition comprising 6-OH CBDV, wherein 85 wt% or more of the 6-OH CBDV is Form E 6-OH CBDV. In a preferred embodiment, the present disclosure provides a composition comprising 6-OH CBDV, wherein 90 wt% or more, more preferably 95 wt% or more, even more preferably 98 wt% or more, of the 6-OH CBDV is Form E 6-OH CBDV.

In one aspect, the present disclosure provides a method for preparing crystalline Form E 6-OH CBDV, the method comprising the following steps:
(a) providing a first vessel comprising 6-OH CBDV and a solvent comprising DMAc and water;
(b) agitating the composition; and
(c) isolating the solids.

The 6-OH CBDV used in the preparation step (step a) may be any form of 6-OH CBDV. Typically, the 6-OH CBDV used in the preparation method is or comprises crystalline 6-OH CBDV, preferably crystalline Form A 6-OH CBDV.

The slurring step (step b) may be carried out using mechanical agitation, such as stirring.

The slurring step (step b) may be carried out at any suitable temperature. Typically, the slurring step is carried out at from 0 °C to 15°C, such as from 3 °C to 10 °C, such as about 5 °C.

In one aspect, the present disclosure provides crystalline Form E 6-OH CBDV prepared by the method outline in paragraphs [0134] to [0137].

### Pharmaceutical Compositions

While it is possible for the crystalline forms A, B, C, D, or E to be administered alone, it is preferable to administer a pharmaceutical composition (e.g., a formulation, preparation, or medicament) comprising crystalline Form A, B, C, D, or E together with one or more other pharmaceutically acceptable ingredients.

Accordingly, the invention provides a pharmaceutical composition comprising a crystalline form of 6-OH CBDV, such as Form A, together with one or more pharmaceutically acceptable ingredients. The present disclosure also provides a pharmaceutical composition comprising crystalline Form B, C, D, or E together with one or more pharmaceutically acceptable ingredients.

In a preferred embodiment, the pharmaceutical composition comprises Form A 6-OH CBDV.

Suitable pharmaceutically acceptable ingredients (e.g. carriers, diluents, excipients, etc.) can be found in standard pharmaceutical texts, for example, Remington: The Science and Practice of Pharmacy, 20th Edition, 2000, pub. Lippincott, Williams & Wilkins; and Handbook of Pharmaceutical Excipients, 9th edition, 2020, pub. Pharmaceutical Press.

Examples of suitable pharmaceutically acceptable ingredients include pharmaceutically acceptable carriers, diluents, excipients, adjuvants, fillers, buffers, binders, disintegrants, preservatives, antioxidants, lubricants, stabilisers, solubilisers, surfactants (e.g., wetting agents), masking agents, colouring agents, flavouring agents, and sweetening agents.

The pharmaceutical composition may be in any suitable form. Examples of suitable forms include tablets (including, e.g., coated tablets), granules, powders, capsules (including, e.g., hard and soft gelatin capsules) and pills.

### Medical Treatment

The Applicant's earlier work, PCT/GB2020/052944, provides data demonstrating the efficacy of 6-OH CBDV in two mouse models of seizure. Specifically, PCT/GB2020/052944 demonstrates that 6-OH CBDV displays anticonvulsant activity in a mouse model of generalised seizure. As such, crystalline forms of 6-OH CBDV, such as Form A, B, C, D or E, as well as pharmaceutical compositions comprising crystalline forms of 6-OH CBDV, will be useful in medical treatment.

Accordingly, the invention provides a crystalline form of 6-OH CBDV, such as Form A, for use in a method of treatment, for example for use in a method of treatment of the human or animal body by therapy (i.e. a method of therapy). The present disclosure also provides crystalline Form B, C, D, or E of 6-OH CBDV for use in a method of treatment, for example for use in a method of treatment of the human or animal body by therapy (i.e. a method of therapy).

The invention also provides a crystalline form of 6-OH CBDV, such as Form A, for use as a medicament. The present disclosure also provides crystalline Form B, C, D, or E of 6-OH CBDV for use as a medicament.

The present disclosure also provides a method of treatment comprising administering to a subject in need of treatment a therapeutically effective amount of a crystalline form of 6-OH CBDV, such as Form A, B, C, D, or E.

The present disclosure also provides the use of crystalline form of 6-OH CBDV, such as Form A, B, C, D, or E, for the manufacture of a medicament.

In preferred embodiments, the crystalline form of 6-OH CBDV is Form A.

### Conditions Treated

The Applicant's earlier work, PCT/GB2020/052944, provides data demonstrating the efficacy of 6-OH CBDV in a mouse model of generalised seizure. As such, crystalline forms of 6-OH CBDV, such as Form A, B, C, D or E, as well as pharmaceutical compositions comprising crystalline forms of 6-OH CBDV, will be useful in the treatment of certain conditions associated with seizure.

Similarly, crystalline forms of 6-OH CBDV, such as Form A, B, C, D or E, as well as pharmaceutical compositions comprising crystalline forms of 6-OH CBDV, will be useful as medicaments for treating (and in the manufacture of medicaments for treating) certain conditions associated with seizure.

In a preferred embodiment, the condition associated with seizure is epilepsy.

In one embodiment, the condition associated with seizure is generalised seizure, such as generalised seizure associated with epilepsy.

In one embodiment, the condition associated with seizure is tonic-clonic seizures, such as tonic-clonic seizures associated with epilepsy.

### Subject/Patient

The method of treatment typically comprises administering a crystalline form of 6-OH CBDV, such as Form A, B, C, D or E, to a subject or patient.

The subject/patient may be a chordate, a vertebrate, a mammal, a placental mammal, a marsupial (e.g., kangaroo, wombat), a rodent (e.g., a guinea pig, a hamster, a rat, a mouse), murine (e.g., a mouse), a lagomorph (e.g., a rabbit), avian (e.g., a bird), canine (e.g., a dog), feline (e.g., a cat), equine (e.g., a horse), porcine (e.g., a pig), ovine (e.g., a sheep), bovine (e.g., a cow), a primate, simian (e.g., a monkey or ape), a monkey (e.g., marmoset, baboon), an ape (e.g., gorilla, chimpanzee, orang-utan, gibbon), or a human.

The subject/patient may be any of its forms of development, for example, the subject/patient may be an infant or child.

In a preferred embodiment, the subject/patient is a human, more preferably an adult human.

The subject/patient may also be a non-human mammal used in laboratory research, such as a rodent. Rodents include rats, mice, guinea pigs and chinchillas.

### Routes of Administration

The method of treatment may comprise administering a crystalline form of 6-OH CBDV, such as Form A, B, C, D or E, to a subject by any convenient route of administration, whether systemically/peripherally or topically (i.e., at the site of desired action).

The route of administration may be oral (e.g., by ingestion).

### Dosages

The method of treatment typically comprises administering a therapeutically effective amount of administering a crystalline form of 6-OH CBDV, such as Form A, B, C, D or E, to a subject.

Appropriate dosages of the crystalline form of 6-OH CBDV, such as Form A, B, C, D or E, as well as pharmaceutical compositions comprising the crystalline form of 6-OH CBDV, such as Form A, B, C, D or E, can vary from patient to patient. Determining the optimal dosage will generally involve balancing the level of therapeutic benefit against any risk or deleterious side effects. The selected dosage level will depend on a variety of factors including, but not limited to, the activity of the particular crystalline form of 6-OH CBDV, the route of administration, the time of administration, the rate of excretion of the compound, the duration of the treatment, other active agents, compounds, and/or materials used in combination, the severity of the condition, and the species, sex, age, weight, condition, general health, and prior medical history of the patient. The dosage and route of administration will ultimately be at the discretion of the clinician, although generally the dosage will be selected to achieve local concentrations at the site of action which achieve the desired effect without causing substantial harmful or deleterious side-effects.

Administration can be effected in one dose, continuously or intermittently (e.g., in divided doses at appropriate intervals) throughout the course of treatment. Single or multiple administrations can be carried out with the dose level and pattern being selected by the treating clinician.

### Other Aspects and Embodiments

Each and every compatible combination of the embodiments described above is explicitly discloses herein, as if each and every combination was individually and explicitly recited.

Carious further aspects and embodiment of the present invention will be apparent to those skilled in the arti in view of the present disclosure.

Where used, "and/or" is to be taken as a specific disclosure of each of the relevant components or features alone as well as a specific disclosure of the combination of the components or features. For example, "A and/or B" is to be taken as specific disclosure of each of i) A, ii) B, and ii) A and B, just as if each were set out individually.

Unless context dictates otherwise, the descriptions and definitions of the features set out above are not limited to any particular aspect or embodiment of the invention and apply equally to all aspects ad embodiments which are described.

### Definitions

The following definitions are provided are provided in order to aid understanding of the invention.

Aw is water activity.

Epilepsy is considered to be a disease of the brain defined by any of the following conditions: (1) At least two unprovoked (or reflex) seizures occurring >24 h apart; (2) one unprovoked (or reflex) seizure and a probability of further seizures similar to the general recurrence risk (at least 60%) after two unprovoked seizures, occurring over the next 10 years; (3) diagnosis of an epilepsy syndrome (A practical clinical definition of epilepsy by the International League Against Epilepsy (ILAE), 2014).

The term "generalized seizure" ("generalized onset seizures") refers to seizures conceptualized as originating at some point within the brain and rapidly engaging bilaterally distributed networks (Operational Classification of Seizure Types by the ILAE, 2017).

The term "pharmaceutically acceptable" pertains to compounds, ingredients, materials, compositions, dosage forms, etc., which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each ingredient (e.g. carrier, diluent, excipient, etc.) must also be "acceptable" in the sense of being compatible with the other ingredients of the composition.

The term "therapeutically-effective amount" pertains to that amount of a compound, or a material, composition or dosage form comprising a compound, which is effective for producing some desired therapeutic effect, commensurate with a reasonable benefit/risk ratio, when administered in accordance with a desired treatment regimen.

A "tonic-clonic seizure" occurs in two phases, a tonic phase typically involving muscle stiffening and loss of consciousness, and a clonic phase typically involving rhythmically jerking of the limbs.

### WORKED EXAMPLES

Certain aspects and embodiments of the invention will not be illustrated by way of example and with reference to the figures described above.

### Analytical Methods

### X-ray Powder Diffraction (XRPD)

XRPD analyses were performed using a Panalytical Empyrean diffractometer equipped with a Cu X-ray tube and a PIXcel 1 D-Medipix3 detector system. The samples were analysed at ambient temperature in transmission mode and held between low density PVC films. The XRPD program parameters were: range 3-40 °2θ or 4-40 °2θ, step size 0.01313°, counting time 23sec, ~5 min run time. Samples were spun at 60 rpm during data collection. XRPD patterns were sorted, manipulated using HighScore Plus v4.9 software.

### Differential Scanning Calorimetry (DSC)

DSC analyses were carried out on a Perkin Elmer Jade Differential Scanning Calorimeter. Accurately weighed samples were placed in crimped aluminium pans (i.e. closed but not gas tight). Each sample was heated under nitrogen at a rate of 10°C/minute to a maximum of 235°C. Indium metal was used as the calibration standard. Temperatures were reported at the transition onset to the nearest 0.01 degree.

### Hyper Differential Scanning Calorimetry (Hyper DSC)

Hyper DSC analyses were carried out on a Perkin Elmer DSC8500 Differential Scanning Calorimeter. Accurately weighed samples were placed in crimped aluminium pans (i.e. closed but not gas tight). Each sample was heated and cooled under nitrogen at a rate of 300°C/minute using a set temperature range of -50 to 180°C. Indium metal was used as the calibration standard.

### Thermogravimetric Differential Thermal Analysis (TG/DTA)

Thermogravimetric analyses were carried out on a Mettler Toledo TGA/DSC1 STARe. The calibration standards were indium and tin. Samples were placed in an aluminium sample pan, inserted into the TG furnace and accurately weighed. Under a stream of nitrogen at a rate of 10°C/minute, the heat flow signal was stabilised for one minute at 30°C, prior to heating to 300°C.

### Nuclear Magnetic Resonance spectroscopy (NMR)

NMR analysis was carried out on a Bruker 500MHz instrument in d6-DMSO. Instrumental parameters are listed on the relevant spectrum plots.

### Optical Microscopy

Microscopy analyses were carried out using an Olympus BX51 stereomicroscope with cross-polarised light and a 1st order red compensator plate. Photomicrographic images were captured using a ColorView Illu digital camera and SynchronizlR basic V5.0 imaging software with objective lens magnification of x10.

### Example 1: Preparation of amorphous 6-OH CBDV

6-OH CBDV was prepared as described in PCT/GB2020/052944.

To CBDV (5.00 g, 17.5 mmol) in anhydrous pyridine (20 mL) was added acetic anhydride (5.63 g, 5.20 mL, 55.2 mmol) and the solution was stirred for 4 h. Dichloromethane (300 mL) was added and the solution was washed with water (200 ml), 1M hydrochloric acid (200 mL), saturated aqueous sodium bicarbonate (200 mL), dried (MgSO4) and concentrated to give CBDV diacetate (6.84 g, quantitative), as a yellow oil which was used without further purification.

To CBDV diacetate (4.00 g, 10.8 mmol) in glacial acetic acid (9 mL) and acetic anhydride (4.96 g, 4.59 mL, 48.6 mmol) was added sodium dichromate (3.86 g, 13.0 mmol) and the mixture was stirred at room temperature for 4 days. The resulting solution was diluted with water (150 mL) and extracted with diethyl ether (2 × 150 mL). The combined organic extracts were washed with saturated aqueous sodium bicarbonate (150 mL), dried (MgSO4) and concentrated to give a yellow oil that was purified using a Biotage Isolera automated chromatography system under normal phase conditions (silica column, gradient of 5 → 33 % ethyl acetate in petrol) with detection at 254 nm to give 6-oxo-CBDV diacetate (1.40 g 33 %), as a colourless oil. (Rf = 0.36 (ethyl acetate - petrol, 1 : 4 v/v))

To lithium aluminium hydride (0.58 g, 15.3 mmol) in diethyl ether (50 mL) at 0 °C was added 6-oxo-CBDV diacetate (1.40 g, 3.64 mmol) in diethyl ether (23 mL) and the mixture was stirred at room temperature for 4 h. The resulting mixture was cooled in an ice bath and cautiously quenched with iced water (100 mL). 1 M Hydrochloric acid (60 mL) was added and the mixture was extracted with diethyl ether (100 mL + 50 mL). The combined organic layers were washed with saturated brine (100 mL), dried (MgSO4) and concentrated to give a pale yellow oil that was purified using a Biotage Isolera automated chromatography system under normal phase conditions (silica column, gradient of 7 → 53 % ethyl acetate in petrol) with detection at 254 nm to give 6-OH-CBDV (0.70 g, 64 %), as a white glassy solid. (Rf = 0.29 (ethyl acetate - petrol, 3 : 7 v/v)).

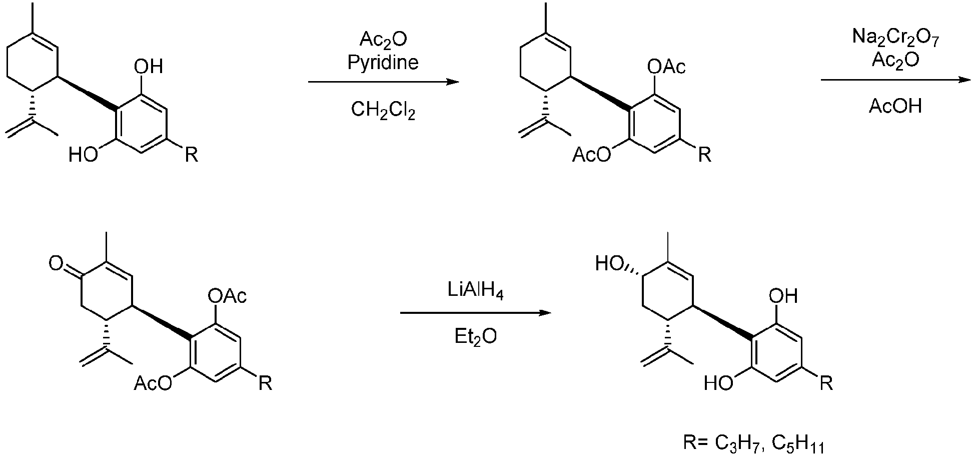

### Characterisation of amorphous 6-OH CBDV

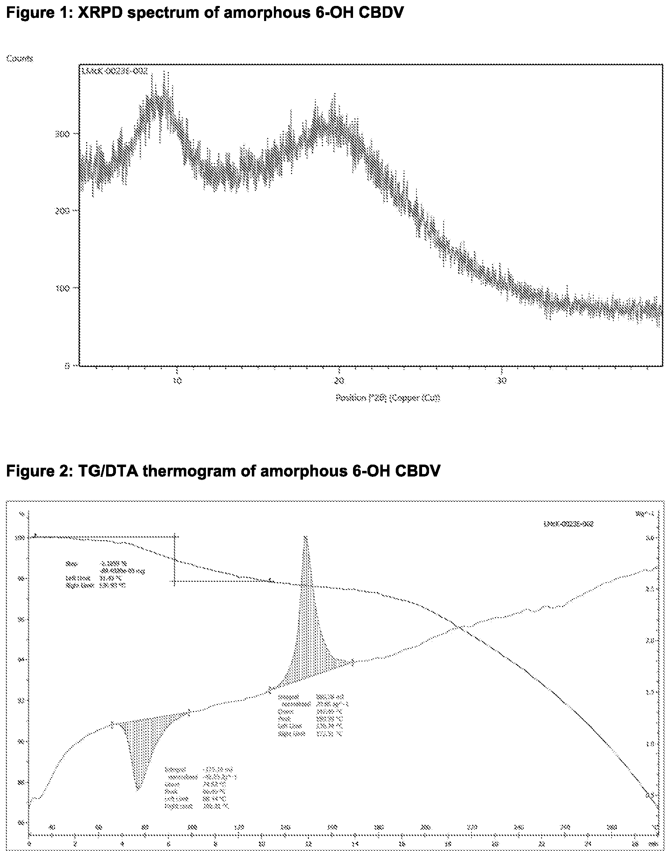

The XRPD pattern obtained for amorphous 6-OH CBDV is shown in Figure 1. The XRPD pattern is indicative of an amorphous material.

A sample of amorphous 6-OH CBDV was analysed by ¹H NMR in d₆-DMSO. The sample contained ~0.15 mol eq. EtOH.

Thermogravimetric/Differential Thermal Analysis (TG/DTA) was performed to determine the thermal profile and associated % weight changes of amorphous 6-OH CBDV.

The thermogram is shown in Figure 2. A weight loss of ∼2.2% was observed between ~31°C and ~137°C, which could account for up to ~0.1 mol eq. water or ~0.4mol eq. EtOH. An exotherm was observed with an onset temperature of ~75°C, followed by and endotherm with an onset temperature of ~148°C.

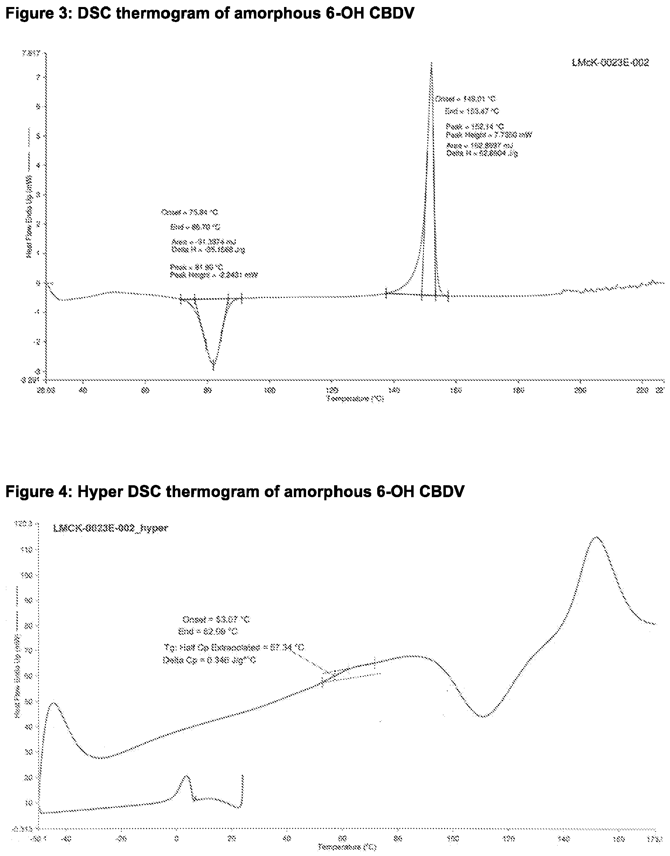

The DSC thermogram shown in Figure 3. An exotherm with an onset temperature of ~76°C can be seen, followed by a sharp endotherm with an onset temperature of ~149°C. The data suggest amorphous material may be crystallising at ~76°C followed by the melt of the crystalline material.

Hyper DSC analysis was carried out on a sample of amorphous 6-OH CBDV. The thermogram is shown in Figure 4, and the temperature of glass transition (T_{g}) was determined to be ~57°C.

Polarised light microscopy showed that the material was composed of a glassy solid. No birefringence was observed. The photomicrograph is shown in Figure 5.

### Example 2: Preparation of Crystalline Form A 6-OH CBDV by Temperature Cycling

Aliquots of the test solvent were added to an accurately weighed sample (-15mg) of amorphous 6-OH-CBDV at ambient temperature. The aliquot volumes were typically 20-100 µL. After the last aliquot of solvent was added (to approx. 40 volumes of solvent), the sample was subjected to two cycles of the following temperature cycling regime:
- Heat from 20°C to within 3°C of solvent boiling point (or 100°C, whichever was lower) at 0.5°C/minute;
- Cool to 20°C at 0.2°C/minute;
- Stirrer speed 800 rpm.

From the infrared (IR) transmission data of the sample vials, dissolution and precipitation events were recorded as the point of complete transmission of IR and the onset of turbidity by IR respectively. Samples were held at ambient temperature for 18 hours to maximise the chance of precipitation. Any recoverable solids were analysed by XRPD.

The experiments are detailed in Table 6. All solids recovered were found to be composed of crystalline Form A material by XRPD.

**Table 1: Results of temperature cycling experiments**

| **Input Form** | **Solvent** | **Result** | **Output Form** |
|---|---|---|---|
| amorphous | heptane | solid | A |
| amorphous | pentane | solid | A |
| amorphous | toluene | solution | N/A |
| amorphous | water | solid | A |
| amorphous | acetone/water (50:50 %v.v.; Aw~0.91) | solution | N/A |

### Characterisation of 6-OH CBDV Form A

The XRPD pattern obtained for crystalline Form A 6-OH-CBDV, is shown in Figure 6. The XRPD pattern is indicative of a crystalline material, however, the slightly elevated baseline may be indicative of some amorphous content. The XRPD peak listing data is given in Table 2.

**Table 2: Observed XRPD data for 6-OH CBDV Form A**

| **No.** | **Pos. [°2θ]** | **Area [cts*°2θ]** | **Backgr.[cts]** | **d-spacing [Å]** | **Height [cts]** | **Rel. Int. [%]** |
|---|---|---|---|---|---|---|
| **1** | 7.83 | 33.00 | 130.61 | 11.29 | 653.760 | 67.3 |
| **2** | 9.26 | 49.04 | 118.03 | 9.56 | 971.490 | 100.0 |
| **3** | 9.55 | 6.77 | 116.09 | 9.26 | 89.400 | 9.2 |
| **4** | 10.75 | 35.03 | 102.05 | 8.23 | 693.990 | 71.4 |
| **5** | 11.24 | 19.48 | 103.14 | 7.87 | 385.900 | 39.7 |
| **6** | 11.39 | 4.66 | 103.40 | 7.77 | 92.390 | 9.5 |
| **7** | 11.68 | 3.90 | 103.49 | 7.57 | 51.500 | 5.3 |
| **8** | 12.03 | 21.81 | 102.90 | 7.36 | 345.630 | 35.6 |
| **9** | 12.50 | 27.95 | 99.99 | 7.08 | 442.980 | 45.6 |
| **10** | 13.01 | 25.70 | 93.01 | 6.80 | 407.260 | 41.9 |
| **11** | 14.64 | 25.92 | 89.68 | 6.05 | 513.480 | 52.9 |
| **12** | 14.93 | 6.90 | 90.19 | 5.93 | 68.350 | 7.0 |
| **13** | 16.36 | 7.76 | 96.31 | 5.42 | 76.840 | 7.9 |
| **14** | 17.28 | 5.68 | 103.91 | 5.13 | 150.130 | 15.5 |
| **15** | 17.52 | 58.21 | 103.93 | 5.06 | 922.580 | 95.0 |
| **16** | 18.30 | 32.22 | 106.34 | 4.85 | 510.650 | 52.6 |
| **17** | 18.52 | 49.67 | 106.62 | 4.79 | 787.180 | 81.0 |
| **18** | 19.31 | 10.66 | 111.46 | 4.60 | 211.240 | 21.7 |
| **19** | 19.94 | 44.26 | 110.77 | 4.45 | 701.500 | 72.2 |
| **20** | 20.79 | 16.18 | 98.00 | 4.27 | 183.160 | 18.9 |
| **21** | 21.34 | 4.05 | 95.95 | 4.16 | 53.500 | 5.5 |
| **22** | 21.60 | 4.95 | 95.33 | 4.11 | 65.330 | 6.7 |
| **23** | 22.62 | 6.77 | 92.84 | 3.93 | 89.460 | 9.2 |
| **24** | 22.85 | 14.51 | 91.50 | 3.89 | 143.710 | 14.8 |
| **25** | 23.53 | 7.06 | 95.85 | 3.78 | 93.220 | 9.6 |
| **26** | 23.70 | 16.85 | 97.33 | 3.75 | 333.780 | 34.4 |
| **27** | 23.90 | 10.14 | 98.39 | 3.72 | 200.960 | 20.7 |
| **28** | 24.08 | 6.41 | 98.59 | 3.70 | 169.400 | 17.4 |
| **29** | 24.60 | 7.95 | 96.02 | 3.62 | 157.470 | 16.2 |
| **30** | 25.43 | 7.23 | 84.26 | 3.50 | 191.010 | 19.7 |
| **31** | 26.22 | 5.72 | 74.19 | 3.40 | 150.970 | 15.5 |
| **32** | 27.95 | 4.24 | 60.23 | 3.19 | 112.120 | 11.5 |
| **33** | 29.24 | 5.55 | 54.02 | 3.05 | 55.010 | 5.7 |
| **34** | 29.59 | 2.64 | 53.04 | 3.02 | 34.860 | 3.6 |
| **35** | 31.40 | 4.91 | 46.37 | 2.85 | 64.870 | 6.7 |

Crystalline Form A 6-OH-CBDV was analysed by ¹H NMR spectroscopy in d₆-DMSO. The spectrum was concordant with the molecular structure. No residual solvent was observed in the spectrum.

Crystalline Form A 6-OH-CBDV was analysed by TG/DTA. The thermogram is shown in Figure 7. A weight loss of ~0.2% was observed between ~33°C and ~132°C, which may be due to loss of surface solvent or water. An endotherm was observed with an onset temperature of ~150°C, which is likely due to the melt.

Crystalline Form A 6-OH-CBDV was analysed by DSC. The thermogram is shown in Figure 8 which shows a single sharp endotherm with an onset temperature of ~151°C.

Photomicrographic images of crystalline Form A 6-OH-CBDV were collected under cross-polarised light. The photomicrograph showed that the material was composed of rod like crystals (Figure 9).

Based on this analysis, it is likely that crystalline Form A material is anhydrous.

### Stability of 6-OH CBDV Form A to Compression

Crystalline Form A material (~60mg) was added to a KBr pellet die and compressed overnight at ~370MPa using a hydraulic press. The resultant solid disc was removed from the press and immediately analysed by XRPD. The resulting diffractogram (Figure 10) showed that the material remained as Form A, indicating good stability to compression, however broader diffraction peaks showed that some disorder was present.

### Stability of Form A to Milling

A ball mill was used to mimic the effects of grinding that would be experienced during formulation steps such as dry blending and wet granulation.

Approximately 45mg of crystalline Form A material was added to a milling chamber with an agate milling ball. Using a Retsch MM200 mixer mill, the material was milled for 6 minutes at a frequency of 25Hz. Periodically, the milling was stopped and powder that adhered to the milling chamber was scraped down. The resultant milled material was analysed using XRPD. Samples were also milled with addition of a small amount of solvent (~10µL).

Form A 6-OH CBDV showed no change in form when ball milled dry or in the presence of a small amount of water, however, the material was more disordered.

**Table 3: Results of milling experiments**

| **Input Form** | **Solvent** | **Screen method** | **Result** | **Output Form** |
|---|---|---|---|---|
| Crystalline | None | dry ball milling | solid | A |
| Crystalline | Water | wet ball milling | solid | A |

### Example 3: Preparation of 6-OH CBDV Form B by Slow Evaporation

Amorphous 6-OH-CBDV (~30mg) was weighed into a virgin glass vial. MEK (140uL) was added, and the resulting solution was left to evaporate in the fume-hood at ambient temperature (~20 °C) in a vial covered with perforated aluminium foil. The recovered solids were analysed by XRPD which confirmed that it was composed of crystalline Form B material.

### Characterisation of 6-OH CBDV Form B

The XRPD pattern obtained for crystalline Form B 6-OH-CBDV, is shown in Figure 11. The XRPD pattern is indicative of a crystalline material. The XRPD peak listing data is given in Table 4.

**Table 4: Observed XRPD data for 6-OH CBDV Form B**

| **No.** | **Pos. [°2θ]** | **Area [cts*°20]** | **Backgr.[cts]** | **d-spacing [Å]** | **Height [cts]** | **Rel. Int. [%]** |
|---|---|---|---|---|---|---|
| **1** | 9.11 | 2.72 | 25.45 | 9.71 | 35.88 | 7.8 |
| **2** | 9.44 | 7.24 | 25.09 | 9.37 | 143.47 | 31.0 |
| **3** | 10.10 | 23.36 | 22.96 | 8.75 | 462.84 | 100.0 |
| **4** | 11.72 | 3.82 | 19.94 | 7.55 | 25.25 | 5.5 |
| **5** | 13.95 | 12.66 | 20.53 | 6.35 | 200.58 | 43.3 |
| **6** | 15.58 | 14.18 | 22.50 | 5.69 | 281.00 | 60.7 |
| **7** | 15.89 | 12.52 | 22.64 | 5.58 | 248.12 | 53.6 |
| **8** | 16.31 | 3.93 | 21.85 | 5.44 | 77.90 | 16.8 |
| **9** | 16.82 | 3.84 | 20.75 | 5.27 | 60.94 | 13.2 |
| **10** | 17.33 | 7.57 | 20.77 | 5.12 | 99.93 | 21.6 |
| **11** | 18.95 | 3.46 | 22.97 | 4.68 | 68.50 | 14.8 |
| **12** | 19.26 | 6.85 | 23.30 | 4.61 | 90.48 | 19.6 |
| **13** | 20.19 | 17.36 | 21.47 | 4.40 | 275.14 | 59.4 |
| **14** | 20.51 | 8.73 | 20.67 | 4.33 | 98.85 | 21.4 |
| **15** | 21.17 | 17.92 | 20.10 | 4.20 | 354.93 | 76.7 |
| **16** | 21.76 | 10.50 | 20.65 | 4.08 | 138.62 | 30.0 |
| **17** | 22.21 | 1.56 | 20.49 | 4.00 | 20.60 | 4.5 |
| **18** | 23.58 | 1.55 | 22.32 | 3.77 | 20.53 | 4.4 |
| **19** | 23.84 | 5.20 | 22.66 | 3.73 | 103.01 | 22.3 |
| **20** | 24.23 | 4.54 | 22.27 | 3.67 | 119.93 | 25.9 |
| **21** | 25.23 | 2.52 | 18.61 | 3.53 | 33.24 | 7.2 |
| **22** | 26.21 | 6.90 | 16.75 | 3.40 | 68.33 | 14.8 |
| **23** | 27.13 | 2.74 | 14.54 | 3.29 | 36.16 | 7.8 |
| **24** | 28.61 | 3.38 | 13.41 | 3.12 | 44.59 | 9.6 |
| **25** | 28.89 | 2.54 | 12.61 | 3.09 | 67.09 | 14.5 |
| **26** | 29.65 | 3.11 | 10.42 | 3.01 | 15.39 | 3.3 |
| **27** | 31.41 | 4.70 | 10.36 | 2.85 | 11.63 | 2.5 |
| **28** | 32.95 | 3.80 | 10.20 | 2.72 | 18.84 | 4.1 |
| **29** | 33.57 | 2.75 | 9.19 | 2.67 | 18.16 | 3.9 |
| **30** | 35.64 | 1.98 | 9.64 | 2.52 | 13.08 | 2.8 |
| **31** | 39.23 | 2.29 | 9.55 | 2.30 | 15.10 | 3.3 |

Crystalline Form B 6-OH-CBDV was analysed by ¹H NMR spectroscopy in d₆-DMSO. The ¹H NMR spectrum of the material conformed to the molecular structure and contained MEK (~1.0 mol eq).

Crystalline Form B 6-OH CBDV was dried under vacuum for ~2hr. The dried sample was analysed by TG/DTA. The thermogram is shown in Figure 12. An endotherm with an onset temperature of ~78°C was noted with a corresponding weight loss of ~15.7% which could account for ~3.1mol eq water or ~0.8mol eq MEK. A second thermogram with an onset temperature of ~147°C was observed.

Based on this analysis, it is likely that crystalline Form B material is an MEK mono-solvate (1:1).

### Example 4: Preparation of 6-OH CBDV Form C by Vapour Stress

X-ray amorphous 6-OH-CBDV (~30mg) was weighed into a virgin glass vial. The vial was placed inside a larger vial containing acetone/water (20:80% v/v,1mL). The larger vial was sealed and left at room temperature (~20 °C) for 7 days. The solids were recovered and analysed by XRPD. The resulting diffractogram confirmed that the recovered solids were composed of crystalline Form C material.

### Characterisation of 6-OH CBDV Form C

The XRPD pattern obtained for crystalline Form C 6-OH-CBDV, is shown in Figure 13. The XRPD pattern is indicative of a crystalline material. The XRPD peak listing data is given in Table 5.

**Table 5: Observed XRPD data for 6-OH CBDV Form C**

| **No.** | **Pos. [°2θ]** | **Area [cts*°20]** | **Backgr.[cts]** | **d-spacing [Å]** | **Height [cts]** | **Rel. Int. [%]** |
|---|---|---|---|---|---|---|
| **1** | 9.21 | 10.31 | 24.36 | 9.60 | 204.35 | 52.6 |
| **2** | 9.59 | 5.97 | 23.36 | 9.22 | 118.28 | 30.5 |
| **3** | 10.20 | 23.93 | 20.57 | 8.67 | 379.34 | 97.7 |
| **4** | 11.87 | 2.26 | 19.27 | 7.46 | 29.79 | 7.7 |
| **5** | 12.08 | 2.19 | 19.36 | 7.33 | 28.91 | 7.4 |
| **6** | 13.96 | 3.49 | 18.05 | 6.34 | 69.12 | 17.8 |
| **7** | 14.22 | 16.20 | 17.42 | 6.23 | 256.74 | 66.1 |
| **8** | 15.85 | 29.41 | 21.42 | 5.59 | 388.42 | 100.0 |
| **9** | 16.14 | 8.09 | 21.39 | 5.49 | 128.15 | 33.0 |
| **10** | 16.51 | 4.86 | 21.05 | 5.37 | 96.31 | 24.8 |
| **11** | 16.97 | 5.22 | 21.29 | 5.23 | 68.99 | 17.8 |
| **12** | 17.58 | 4.48 | 20.32 | 5.05 | 59.22 | 15.3 |
| **13** | 19.27 | 5.15 | 22.63 | 4.61 | 67.99 | 17.5 |
| **14** | 19.67 | 10.92 | 22.75 | 4.51 | 173.02 | 44.5 |
| **15** | 20.53 | 13.20 | 22.07 | 4.33 | 209.23 | 53.9 |
| **16** | 20.92 | 8.95 | 21.44 | 4.25 | 101.33 | 26.1 |
| **17** | 21.40 | 16.31 | 20.54 | 4.15 | 184.67 | 47.5 |
| **18** | 21.86 | 5.96 | 19.39 | 4.07 | 78.67 | 20.3 |
| **19** | 23.95 | 3.04 | 19.90 | 3.72 | 24.09 | 6.2 |
| **20** | 24.22 | 3.58 | 19.48 | 3.68 | 47.24 | 12.2 |
| **21** | 24.39 | 3.92 | 19.16 | 3.65 | 51.80 | 13.3 |
| **22** | 24.65 | 9.95 | 18.31 | 3.61 | 98.56 | 25.4 |
| **23** | 26.59 | 4.23 | 11.27 | 3.35 | 47.92 | 12.3 |
| **24** | 27.54 | 2.96 | 10.81 | 3.24 | 19.53 | 5.0 |
| **25** | 28.68 | 2.41 | 12.26 | 3.11 | 19.11 | 4.9 |
| **26** | 28.96 | 3.45 | 12.24 | 3.08 | 27.37 | 7.1 |
| **27** | 29.32 | 5.76 | 11.76 | 3.05 | 38.02 | 9.8 |
| **28** | 31.65 | 2.65 | 10.28 | 2.83 | 17.50 | 4.5 |
| **29** | 33.41 | 3.11 | 8.99 | 2.68 | 12.31 | 3.2 |
| **30** | 34.17 | 2.11 | 7.29 | 2.62 | 13.92 | 3.6 |
| **31** | 38.33 | 2.91 | 7.40 | 2.35 | 9.61 | 2.5 |

Crystalline Form C 6-OH-CBDV was analysed by ¹H NMR spectroscopy in d₆-DMSO. The ¹H NMR spectrum of the material conformed to the molecular structure and contained acetone (~1.0 mol eq).

Crystalline Form C 6-OH-CBDV was analysed by TG/DTA. The thermogram is shown in Figure 14. The TG/DTA thermogram showed an endotherm with an onset temperature of ~80°C. A corresponding weight loss of ~16.3% (equivalent to ~1.0 mol eq. acetone) was observed between ~30-125°C and is likely due to loss of solvent. A second endotherm with an onset temperature of ~148°C likely to be due to the melt.

Based on this analysis, it is likely that crystalline Form C material is an acetone mono-solvate (1:1).

### Example 5: Preparation of 6-OH CBDV Form D by Sonication

Crystalline Form A 6-OH-CBDV (~30mg) was weighed into a virgin glass vial. NMP/water (10:90%v/v, 50uL), was added and the mixture was sonicated sonicated at 70% intensity using a Cole-Parmer 130W ultrasonic processor for 3 minutes in 30 second intervals. The solids were recovered and analysed by XRPD. The diffractogram confirmed that the solids were composed of crystalline Form D material.

### Characterisation of 6-OH CBDV Form D

The XRPD pattern obtained for crystalline Form D 6-OH-CBDV, is shown in Figure 15. The XRPD pattern is indicative of a crystalline material. The XRPD peak listing data is given in Table 6.

**Table 6: Observed XRPD data for 6-OH CBDV Form D**

| **No.** | **Pos. [°2θ]** | **Area [cts*°2θ]** | **Backgr.[cts]** | **d-spacing [Å]** | **Height [cts]** | **Rel. Int. [%]** |
|---|---|---|---|---|---|---|
| **1** | 7.82 | 3.81 | 26.15 | 11.31 | 50.32 | 10.5 |
| **2** | 8.16 | 5.04 | 24.76 | 10.84 | 99.89 | 20.7 |
| **3** | 9.25 | 4.28 | 21.46 | 9.56 | 84.88 | 17.6 |
| **4** | 10.34 | 4.22 | 21.06 | 8.56 | 66.88 | 13.9 |
| **5** | 10.73 | 7.91 | 21.43 | 8.24 | 125.30 | 26.0 |
| **6** | 10.85 | 5.93 | 21.53 | 8.15 | 117.41 | 24.4 |
| **7** | 11.23 | 3.02 | 21.65 | 7.88 | 39.88 | 8.3 |
| **8** | 12.00 | 5.35 | 20.99 | 7.37 | 70.67 | 14.7 |
| **9** | 12.46 | 10.29 | 20.74 | 7.10 | 135.92 | 28.2 |
| **10** | 13.01 | 2.91 | 20.18 | 6.81 | 46.18 | 9.6 |
| **11** | 14.00 | 2.47 | 18.83 | 6.33 | 32.67 | 6.8 |
| **12** | 14.63 | 2.30 | 18.60 | 6.05 | 30.36 | 6.3 |
| **13** | 15.28 | 36.46 | 18.99 | 5.80 | 481.51 | 100.0 |
| **14** | 16.05 | 2.80 | 19.07 | 5.52 | 36.94 | 7.7 |
| **15** | 17.65 | 30.35 | 22.85 | 5.03 | 400.92 | 83.3 |
| **16** | 18.56 | 12.21 | 23.53 | 4.78 | 96.73 | 20.1 |
| **17** | 19.19 | 8.67 | 23.11 | 4.62 | 114.49 | 23.8 |
| **18** | 19.56 | 7.60 | 22.50 | 4.54 | 120.38 | 25.0 |
| **19** | 19.93 | 2.86 | 21.62 | 4.45 | 37.72 | 7.8 |
| **20** | 20.78 | 3.45 | 24.56 | 4.28 | 34.19 | 7.1 |
| **21** | 21.16 | 5.98 | 25.78 | 4.20 | 59.20 | 12.3 |
| **22** | 21.84 | 3.95 | 27.21 | 4.07 | 39.17 | 8.1 |
| **23** | 22.24 | 10.02 | 27.74 | 4.00 | 79.40 | 16.5 |
| **24** | 22.71 | 3.81 | 27.93 | 3.92 | 25.14 | 5.2 |
| **25** | 23.19 | 6.85 | 27.68 | 3.84 | 54.25 | 11.3 |
| **26** | 23.61 | 4.69 | 27.26 | 3.77 | 23.25 | 4.8 |
| **27** | 24.60 | 4.53 | 25.55 | 3.62 | 44.89 | 9.3 |
| **28** | 26.22 | 5.96 | 23.95 | 3.40 | 29.51 | 6.1 |
| **29** | 26.77 | 6.25 | 24.46 | 3.33 | 82.60 | 17.2 |
| **30** | 27.89 | 4.41 | 23.93 | 3.20 | 29.11 | 6.0 |
| **31** | 31.42 | 3.99 | 17.56 | 2.85 | 26.33 | 5.5 |
| **32** | 38.29 | 2.73 | 12.85 | 2.35 | 13.52 | 2.8 |

Crystalline Form D 6-OH-CBDV was analysed by ¹H NMR spectroscopy in d₆-DMSO. The ¹H NMR spectrum of the material conformed to the molecular structure and contained NMP (~0.8 mol eq).

Crystalline Form D 6-OH-CBDV was analysed by TG/DTA. The thermogram is shown in Figure 16. The thermogram showed a weight loss of ~1.2% between ~30-65°C, likely due to surface moisture/solvent. A further weight loss of ~33% was observed between ~66-142°C, which corresponded to a broad endotherm with an onset temperature of ~94°C. This could be the melt of material, or dissolution of the material in hot NMP. The second weight loss could account for up to ~1.5mol eq NMP or ~8.3mol eq water and may be due to a loss of a mixture of both NMP and water.

Crystalline Form D may be an NMP solvate or a hydrate.

### Example 6: Preparation of 6-OH CBDV Form E

Crystalline Form A 6-OH-CBDV (~30mg) was weighed into a virgin glass vial. DMAc/water (10:90%v/v, 100uL) was added, the vial was sealed, and the resulting slurry was stirred at 5°C for 7 days. The solids were isolated by centrifugation and dried under flow of air prior to analysis by XRPD, which confirmed that the solid was composed of crystalline Form E material.

### Characterisation of 6-OH CBDV Form E

The XRPD pattern obtained for crystalline Form E 6-OH-CBDV, is shown in Figure 17. The XRPD pattern is indicative of a crystalline material, but the elevated baseline may be indicative of some disorder or amorphous content. The XRPD peak listing data is given in Table 7.

**Table 7: Observed XRPD data for 6-OH CBDV Form E**

| **No.** | **Pos. [°2θ]** | **Area [cts*°20]** | **Backgr.[cts]** | **d-spacing [Å]** | **Height [cts]** | **Rel. Int. [%]** |
|---|---|---|---|---|---|---|
| **1** | 8.29 | 3.78 | 25.48 | 10.66 | 49.94 | 14.0 |
| **2** | 10.27 | 3.90 | 23.38 | 8.61 | 25.73 | 7.2 |
| **3** | 10.73 | 4.91 | 22.80 | 8.25 | 77.86 | 21.8 |
| **4** | 10.96 | 7.24 | 22.26 | 8.07 | 114.82 | 32.2 |
| **5** | 12.05 | 2.92 | 20.88 | 7.35 | 19.28 | 5.4 |
| **6** | 12.62 | 7.14 | 20.67 | 7.02 | 94.26 | 26.4 |
| **7** | 14.05 | 3.41 | 18.71 | 6.31 | 33.77 | 9.5 |
| **8** | 15.37 | 31.54 | 20.83 | 5.76 | 357.10 | 100.0 |
| **9** | 17.52 | 25.76 | 25.60 | 5.06 | 340.28 | 95.3 |
| **10** | 18.68 | 8.69 | 27.33 | 4.75 | 98.41 | 27.6 |
| **11** | 19.74 | 7.20 | 24.82 | 4.50 | 95.04 | 26.6 |
| **12** | 20.75 | 1.92 | 21.96 | 4.28 | 25.30 | 7.1 |
| **13** | 21.48 | 5.02 | 24.94 | 4.14 | 49.73 | 13.9 |
| **14** | 22.30 | 4.27 | 26.69 | 3.99 | 42.28 | 11.8 |
| **15** | 22.96 | 2.59 | 25.63 | 3.87 | 34.25 | 9.6 |
| **16** | 23.55 | 3.58 | 24.95 | 3.78 | 47.24 | 13.2 |
| **17** | 25.16 | 3.59 | 25.51 | 3.54 | 23.69 | 6.6 |
| **18** | 27.25 | 6.00 | 28.48 | 3.27 | 47.55 | 13.3 |
| **19** | 27.75 | 2.19 | 27.07 | 3.21 | 28.99 | 8.1 |
| **20** | 31.85 | 3.08 | 21.05 | 2.81 | 10.18 | 2.9 |

Crystalline Form E 6-OH-CBDV was analysed by ¹H NMR spectroscopy in d₆-DMSO. The ¹H NMR spectrum of the material conformed to the molecular structure and contained ~1.0 mol eq DMAc.

Crystalline Form E 6-OH-CBDV was analysed by TG/DTA. The thermogram is shown in Figure 18. The TG/DTA thermogram showed a weight loss of ~28% (which could account for up to ~1.4 mol eq DMAc or ~6.6mol eq water) between ~30-143°C. This corresponded to a broad, complex endotherm with an onset temperature of ~98°C. The weight loss may be due to a mixture of water (~0.5mol eq, ~5.6%wt loss) and DMAc (~1mol, ~22.4%wt loss). 6-OH-CBDV Form E may be a mixed DMAc solvate/hydrate.

### Further Characterisation of Form E

A sample of 6-OH-CBDV Form E material was dried under vacuum for ~2hr prior to reanalysis by XRPD (Figure 19). The diffractogram showed that the material was still composed of Pattern E material, however, slight peak shifting was noted.

Dried Pattern E 6-OH-CBDV was analysed by ¹H NMR spectroscopy. The spectrum was concordant with the molecular structure. DMAc (~1.0mol eq) was present in the sample.

A sample of the dried Pattern E material was analysed by TG/DTA. The thermogram is shown in Figure 20 and shows a weight loss of ~2.5% between 45-148°C. The weight loss could account for ~0.4mol eq water or ~0.09mol eq DMAc and may be due to surface bound residual solvent. A second weight loss of ~25.1% was observed between 149-286°C which could account for ~5.6mol eq water or ~1.2mol eq DMAc, however, some of the weight loss is likely due to the onset of decomposition of the material. An endotherm with an onset temperature of ~117°C was observed.

Based upon this data, it is possible that the initially isolated 6-OH-CBDV Form E may be a mixed hydrate:DMAc solvate (0.5:1) and that the dried Form E 6-OH-CBDV is a mono DMAc solvate (1:1).

### REFERENCES

A number of publications are cited above in order to more fully describe and disclose the invention and the state of the art to which the invention pertains. Full citations for these references are provided below.
1. PCT/GB2020/052944

## Claims

1. A crystalline form of 6-hydroxy cannabidivarin (6-OH CBDV).

2. The crystalline form of claim 1 **characterized by** an X-ray powder diffraction (XRPD) pattern comprising peaks at 7.83, 12.50, 13.01, 14.64, 18.30, 20.79, 23.90, 24.60, 25.43, and 26.22 ±0.2 (°2θ) when measured using Cu-K_{α} irradiation (1.54060 Å).

3. The crystalline form of claim 2 **characterized by** a differential scanning calorimetry (DCS) thermogram comprising a peak with an onset temperature (Tₒₙₛₑₜ) of between 142 °C and 153 °C.

4. The crystalline form of claim 3 **characterized by** a DCS thermogram comprising a peak with a Tₒₙₛₑₜ of about 151 °C.

5. The crystalline form of any of claims 2 to 4 **characterised by** rod-like crystals.

6. The crystalline form of any of claims 2 to 5 **characterized by** an XRPD pattern further comprising peaks at 9.26, 10.75, 11.24, 12.03, 17.28, 18.52, 23.70, and 24.08 ±0.2 (°2θ) when measured using Cu-K_{α} irradiation (1.54060 Å).

7. The crystalline form of any of claims 2 to 6 **characterized by** an XRPD pattern further comprising peaks at 17.52, 19.31, and 19.94 ±0.2 (°2θ) when measured using Cu-K_{α} irradiation (1.54060 Å).

8. The crystalline form of any of claims 2 to 7 **characterized by** an XRPD pattern measured using Cu-K_{α} irradiation (1.54060 Å) substantial as shown in Figure 6.

9. A composition comprising 6-OH CBDV, wherein:
(i) 5 wt% or more, such as 50 wt% or more; or
(ii) 90 wt% or more, such as 95 wt% or more;
of the 6-OH CBDV is the crystalline form of any of claims 1 to 8.

10. A pharmaceutical composition comprising the crystalline form of any one of claims 1 to 8, or the composition of claim 9, together with one or more ingredients selected from carriers, diluents, excipients, adjuvants, fillers, buffers, binders, disintegrants, preservatives, antioxidants, lubricants, stabilisers, solubilisers, surfactants, masking agents, colouring agents, flavouring agents, and sweetening agents.

11. The pharmaceutical composition of claim 10 in a form selected from a tablet, a granule, a powder, a capsule, and a pill.

12. The crystalline form of any one of claims 1 to 8, or the composition of any one of claims 9 to 11, for use in a method of treatment, such as in the treatment of a condition associated with seizure, optionally wherein the condition associated with seizure is epilepsy.

13. A method for preparing a crystalline of 6-OH CBDV, the method comprising the following steps:
(a) providing a composition comprising 6-OH CBDV and a solvent selected from water and a hydrocarbon solvent;
(b) heating the composition;
(c) cooling the composition; and
(d) isolating the solids.

14. The method of claim 13, wherein: the solvent is an aliphatic hydrocarbon solvent, optionally wherein the aliphatic hydrocarbon solvent is heptane or pentane; or the solvent is an aromatic hydrocarbon solvent, optionally wherein the aromatic hydrocarbon solvent is toluene.

15. The method of any of claims 13 to 14, wherein:
i. step (b) comprises heating the composition to a predetermined maximum temperature, optionally wherein the predetermined maximum temperature is the lower of:
i) 10 °C lower than the solvent boiling point; or
ii) 100°C;
ii. step (b) comprises heating the composition at a predetermined heating, optionally wherein the predetermined heating rate is from 0.2 °C/min to 20 °C/min;
iii. step (c) comprises cooling the composition to a predetermined minimum temperature, optionally wherein the predetermined minimum temperature is approx.. 20°C;
iv. step (d) comprises isolating the solids by filtration or centrifugation; and/or
v. steps (b) and (c) are repeated.

## Patentansprüche

1. Kristalline Form von 6-Hydroxycannabidivarin (6-OH-CBDV).

2. Kristalline Form nach Anspruch 1, **gekennzeichnet durch** ein Röntgenpulverbeugungs(XRPD-)Muster, das Peaks bei 7,83, 12,50, 13,01, 14,64, 18,30, 20,79, 23,90, 24,60, 25,43 und 26,22±0,2 (°2θ) umfasst, wenn unter Verwendung von Cu-K_{α}-Strahlung (1,54060 Å) gemessen.

3. Kristalline Form nach Anspruch 2, **gekennzeichnet durch** ein Differential-Scanning-Kalorimetrie(DCS-)Thermogramm, das einen Peak mit einer Einsatztemperatur (Tₒₙₛₑₜ) zwischen 142 °C und 153 °C umfasst.

4. Kristalline Form nach Anspruch 3, **gekennzeichnet durch** ein DCS-Thermogramm, das einen Peak mit einer Tₒₙₛₑₜ von etwa 151 °C umfasst.

5. Kristalline Form nach einem der Ansprüche 2 bis 4, **gekennzeichnet durch** stabförmige Kristalle.

6. Kristalline Form nach einem der Ansprüche 2 bis 5, **gekennzeichnet durch** ein XRPD-Muster, das ferner Peaks bei 9,26, 10,75, 11,24, 12,03, 17,28, 18,52, 23,70 und 24,08±0,2 (°2θ) umfasst, wenn unter Verwendung von Cu-K_{α}-Strahlung (1,54060 Å) gemessen.

7. Kristalline Form nach einem der Ansprüche 2 bis 6, **gekennzeichnet durch** ein XRPD-Muster, das ferner Peaks bei 17,52, 19,31 und 19,94±0,2 (°2θ) umfasst, wenn unter Verwendung von Cu-K_{α}-Strahlung (1,54060 Å) gemessen.

8. Kristalline Form nach einem der Ansprüche 2 bis 7, **gekennzeichnet durch** ein XRPD-Muster, gemessen unter Verwendung von Cu-K_{α}-Strahlung (1,54060 Å), im Wesentlichen wie in Figur 6 gezeigt.

9. Zusammensetzung, umfassend 6-OH-CBDV, wobei:
(i) 5 Gew.-% oder mehr, wie etwa 50 Gew.-% oder mehr; oder
(ii) 90 Gew.-% oder mehr, wie etwa 95 Gew.-% oder mehr;
des 6-OH-CBDV die kristalline Form nach einem der Ansprüche 1 bis 8 ist.

10. Pharmazeutische Zusammensetzung, umfassend die kristalline Form nach einem der Ansprüche 1 bis 8 oder Zusammensetzung nach Anspruch 9, zusammen mit einem oder mehreren Inhaltsstoffen, die ausgewählt sind aus Trägern, Verdünnungsmitteln, Hilfsstoffen, Adjuvantien, Füllstoffen, Puffern, Bindemitteln, Zerfallsmitteln, Konservierungsmitteln, Antioxidantien, Schmiermitteln, Stabilisatoren, Lösungsvermittlern, Tensiden, Maskierungsmitteln, Farbstoffen, Aromastoffen und Süßungsmitteln.

11. Pharmazeutische Zusammensetzung nach Anspruch 10 in einer Form, die aus einer Tablette, einem Granulat, einem Pulver, einer Kapsel und einer Pille ausgewählt ist.

12. Kristalline Form nach einem der Ansprüche 1 bis 8 oder Zusammensetzung nach einem der Ansprüche 9 bis 11 zur Verwendung bei einem Behandlungsverfahren, wie bei der Behandlung eines Zustands, der mit einem Anfall assoziiert ist, wobei optional der Zustand, der mit einem Anfall assoziiert ist, Epilepsie ist.

13. Verfahren zur Herstellung eines Kristallins von 6-OH-CBDV, wobei das Verfahren die folgenden Schritte umfasst:
(a) Bereitstellen einer Zusammensetzung, die 6-OH-CBDV und ein Lösungsmittel umfasst, das aus Wasser und einem Kohlenwasserstofflösungsmittel ausgewählt ist;
(b) Erwärmen der Zusammensetzung;
(c) Abkühlen der Zusammensetzung; und
(d) Isolieren der Feststoffe.

14. Verfahren nach Anspruch 13, wobei: das Lösungsmittel ein aliphatisches Kohlenwasserstofflösungsmittel ist, wobei optional das aliphatische Kohlenwasserstofflösungsmittel Heptan oder Pentan ist; oder das Lösungsmittel ein aromatisches Kohlenwasserstofflösungsmittel ist, wobei optional das aromatische Kohlenwasserstofflösungsmittel Toluol ist.

15. Verfahren nach einem der Ansprüche 13 bis 14, wobei:
i. Schritt (b) Erwärmen der Zusammensetzung auf eine vorbestimmte Maximaltemperatur umfasst, wobei optional die vorbestimmte Maximaltemperatur die niedrigere von Folgenden ist:
i) 10 °C niedriger als der Siedepunkt des Lösungsmittels; oder
ii) 100 °C;
ii. Schritt (b) Erwärmen der Zusammensetzung bei einem vorbestimmten Erwärmen umfasst, wobei optional die vorbestimmte Erwärmungsrate von 0,2 °C/min bis 20 °C/min beträgt;
iii. Schritt (c) Abkühlen der Zusammensetzung auf eine vorbestimmte Mindesttemperatur umfasst, wobei optional die vorbestimmte Mindesttemperatur ca. 20 °C beträgt;
iv. Schritt (d) Isolieren der Feststoffe durch Filtration oder Zentrifugation umfasst; und/oder
v. Schritt (b) und (c) wiederholt werden.

## Revendications

1. Forme cristalline de la 6-hydroxy cannabidivarine (6-OH CBDV).

2. Forme cristalline de la revendication 1, **caractérisée par** un diagramme de diffraction des rayons X sur poudre (XRPD) comprenant des pics à 7,83, 12,50, 13,01, 14,64, 18,30, 20,79, 23,90, 24,60, 25,43 et 26,22 ± 0,2 (°2θ) lorsqu'il est mesuré à l'aide d'un rayonnement Cu-K_{α} (1,54060 Å).

3. Forme cristalline de la revendication 2, **caractérisée par** un thermogramme de calorimétrie différentielle à balayage (DSC) comprenant un pic dont la température de début (T_{début}) est comprise entre 142 °C et 153 °C.

4. Forme cristalline de la revendication 3, **caractérisée par** un thermogramme DCS comprenant un pic dont la T_{début} est d'environ 151 °C.

5. Forme cristalline de l'une quelconque des revendications 2 à 4, **caractérisée par** des cristaux en forme de bâtonnets.

6. Forme cristalline de l'une quelconque des revendications 2 à 5, **caractérisée par** un diagramme XRPD comprenant en outre des pics à 9,26, 10,75, 11,24, 12,03, 17,28, 18,52, 23,70 et 24,08 ± 0,2 (°2θ) lorsqu'il est mesuré à l'aide d'un rayonnement Cu-K_{α} (1,54060 Å).

7. Forme cristalline de l'une quelconque des revendications 2 à 6, **caractérisée par** un diagramme XRPD comprenant en outre des pics à 17,52, 19,31 et 19,94 ± 0,2 (°2θ) lorsqu'il est mesuré à l'aide d'un rayonnement Cu-K_{α} (1,54060 Å).

8. Forme cristalline de l'une quelconque des revendications 2 à 7, **caractérisée par** un diagramme XRPD mesuré à l'aide d'un rayonnement Cu-K_{α} (1,54060 Å) sensiblement tel que représenté sur la Figure 6.

9. Composition comprenant la 6-OH CBDV, dans laquelle :
(i) 5 % en poids ou plus, par exemple 50 % en poids ou plus ; ou
(ii) 90 % en poids ou plus, par exemple 95 % en poids ou plus ;
de la 6-OH CBDV est la forme cristalline de l'une quelconque des revendications 1 à 8.

10. Composition pharmaceutique comprenant la forme cristalline de l'une quelconque des revendications 1 à 8, ou composition de la revendication 9, avec un ou plusieurs ingrédients choisis parmi des supports, des diluants, des excipients, des adjuvants, des charges, des tampons, des liants, des délitants, des conservateurs, des antioxydants, des lubrifiants, des stabilisants, des solubilisants, des tensioactifs, des agents masquants, des agents colorants, des agents aromatisants et des agents édulcorants.

11. Composition pharmaceutique de la revendication 10 sous une forme choisie parmi un comprimé, un granulé, une poudre, une capsule et une pilule.

12. Forme cristalline de l'une quelconque des revendications 1 à 8, ou composition de l'une quelconque des revendications 9 à 11, destinée à être utilisée dans un procédé de traitement, tel que dans le traitement d'une maladie associée à des crises, éventuellement dans laquelle la maladie associée à des crises est l'épilepsie.

13. Procédé permettant la préparation d'une cristalline de la 6-OH CBDV, le procédé comprenant les étapes suivantes :
(a) la fourniture d'une composition comprenant la 6-OH CBDV et un solvant choisi parmi l'eau et un solvant hydrocarboné ;
(b) le chauffage de la composition ;
(c) le refroidissement de la composition ; et
(d) l'isolement des solides.

14. Procédé de la revendication 13, dans lequel : le solvant est un solvant hydrocarboné aliphatique, éventuellement dans lequel le solvant hydrocarboné aliphatique est l'heptane ou le pentane ; ou le solvant est un solvant hydrocarboné aromatique, éventuellement dans lequel le solvant hydrocarboné aromatique est le toluène.

15. Procédé de l'une quelconque des revendications 13 à 14, dans lequel :
i. l'étape (b) comprend le chauffage de la composition jusqu'à une température maximale prédéterminée, éventuellement dans lequel la température maximale prédéterminée est la plus basse parmi :
i) une température inférieure de 10 °C au point d'ébullition du solvant ; ou
ii) 100 °C ;
ii. l'étape (b) comprend le chauffage de la composition à un chauffage prédéterminé, éventuellement dans lequel la vitesse de chauffage prédéterminée est comprise entre 0,2 °C/min et 20 °C/min ;
iii. l'étape (c) comprend le refroidissement de la composition jusqu'à une température minimale prédéterminée, éventuellement dans lequel la température minimale prédéterminée est d'environ 20 °C ;
iv. l'étape (d) comprend l'isolement des solides par filtration ou centrifugation ; et/ou
v. les étapes (b) et (c) sont répétées.
